# EUROPEAN PATENT APPLICATION

(11) **EP 4 835 447 A1**
(43) Date of publication of application: **04.11.2026**
(21) Application number: 25173641.9
(22) Date of filing: 30.04.2025
(51) Int. Cl.: G01N 21/64, G01N 21/25, G01N 33/50

(54) **A SYSTEM AND A METHOD FOR SPATIAL ANALYSIS OF A BIOLOGICAL SAMPLE**

(71) Applicant: Imec VZW, 3001 Leuven (BE)
(72) Inventor: HA, Seungkyu, 3001 Heverlee (BE); SET, Ying Ting, 3001 Heverlee (BE); VERELLEN, Niels, 3001 Heverlee (BE); LAGAE, Liesbet, 3000 Leuven (BE); YURT, Abdulkadir, 3360 Korbeek-Lo (BE)
(74) Representative: AWA Sweden AB

(57) **Abstract**

A system (100) for spatial analysis of a biological sample comprises: a functionalized surface (110) comprising a plurality of binding sites (112) of a plurality of binding site types, wherein binding sites (112a-d) of different binding site types are configured to bind different categories of biological entities from the biological sample, wherein the plurality of binding sites (112) comprises a plurality of subsets (114); an illumination system (118) configured to form an illumination pattern for selectively illuminating one or more binding sites (112) at a time; and an array of light-detecting areas (130) configured to detect output light associated with biological entities bound at binding sites (112) and illuminated by the illumination pattern, wherein each light-detecting area (130) is associated with a subset (114); wherein the illumination system (118) and the array of light-detecting areas (130) are configured to differentiate responses to the illumination pattern from different binding sites (112).

## Description

### Technical field

The present description relates to a system and a method for spatial analysis of a biological sample.

### Background

Multi-cellular organisms have evolved to develop complex tissues made of cells that may be organized in intricate spatial patterns, which allow them to perform diverse physiological functions. In their native environment, those cells do not operate by themselves in isolation but rather interact with one another and their surroundings. As the multi-cellular organisms develop, the cells differentiate from one another, taking different specialized roles in the organism. On a larger scale, function and state of tissue are fundamentally tied to the types of cells in the tissue, the spatial arrangements and interactions of the cells. Studying all these aspects therefore provides important information for understanding a biological system as a whole and elucidating how cellular composition down to molecular level contributes to functioning of the biological system.

Traditional sensing and sequencing methods for biological systems have, however, been analyzing cells or molecules in isolation, causing a loss of information about the spatial relationships and interactions of cells in the biological system. In recent years, spatial biology has emerged as a field focusing on understanding spatial organization, distribution, and interactions of molecules, nuclei, cells, and tissues. Preserving spatial context helps scientists in the biosensing field and medical professionals to better understand behaviors, biological functions, and responses to diseases and other physiological conditions of biological systems. For example, spatial biology techniques allow for mapping gene expression, deoxyribonucleic acid (DNA), ribonucleic acid (RNA), aptamers, and other molecular features directly within their spatial context of tissues. The potential is even more promising when considering the high throughput of next-generation sequencing (NGS) technologies that allow for sequencing millions of molecules in parallel, reducing the time required to generate the spatial data.

However, there is a need for providing spatial information for different aspects of biological samples with a high resolution.

### Summary

An objective of the present description is to enable spatial analysis of a biological sample with high spatial resolution. Another objective of the present description is to enable spatial analysis of multiple aspects of the biological sample.

These and other objectives are at least partly met by the invention as defined in the independent claims. Preferred embodiments are set out in the dependent claims.

According to a first aspect, there is provided a system for spatial analysis of a biological sample, said system comprising: a functionalized surface, wherein the functionalized surface is configured to receive the biological sample and comprises a plurality of binding sites of a plurality of binding site types, wherein the binding sites of a binding site type are distributed across the plurality of binding sites and are configured to bind a biological entity from the biological sample, wherein binding sites of different binding site types are configured to bind different categories of biological entities from the biological sample, wherein the plurality of binding sites comprises a plurality of subsets, wherein each subset comprises at least one binding site or part of a binding site; an illumination system arranged in relation to the functionalized surface and configured to form an illumination pattern at the functionalized surface for selectively illuminating one or more binding sites at a time; and an array of light-detecting areas arranged in relation to the functionalized surface and configured to detect output light associated with biological entities bound at binding sites and illuminated by the illumination pattern, wherein each light-detecting area is associated with a subset of the binding sites; wherein the illumination system and the array of light-detecting areas are configured to provide imaging of the functionalized surface for differentiating responses to the illumination pattern from different binding sites.

The system enables determining spatial information of a biological sample with a high resolution. Thanks to using binding sites, biological entities of the biological sample may be bound at a particular spatial location. Each binding site can be associated with a corresponding spatial location in the biological sample by the biological entities from the sample being bound at a binding site closest to an origin of the biological entity. The biological entities may for instance be spatially diffused from the biological sample to the respective binding sites. Thus, the biological entities may be analyzed at the spatial location such that the information acquired of a biological entity bound at the binding site may be related to the corresponding spatial location in the sample.

The binding sites may be provided with a high resolution such that spatial information of the biological sample is also provided with high resolution. Thus, the functionalized surface may be patterned by the binding sites being arranged on the functionalized surface. The system further provides an illumination system and an array of light-detecting areas configured to allow imaging of the binding sites such that the imaging allows differentiating responses from different binding sites. Thus, the illumination system and the array of light-detecting areas ensure that a high resolution patterning of the binding sites can also be converted to high resolution imaging of the biological sample.

The binding sites of different binding site types are configured to bind different categories of biological entities from the biological sample. This implies that multiple aspects of the biological sample may be analyzed using the same functionalized surface. Hence, spatial relationships between different categories of biological entities within the same sample may be determined, allowing the system to provide an insight into organization of complex tissues.

The biological entity may be a molecule, such as a deoxyribonucleic acid (DNA) molecule, a ribonucleic acid (RNA) molecule, a protein, a peptide, a metabolite, a lipid, a sugar, a vitamin, a hormone, an aptamer, a small-molecule drug, or other molecular features. The biological entity may alternatively be a complex entity comprising two or more molecules bound to each other. The biological entity may be a complex entity of an antibody and an antigen, an enzyme and a substrate, a protein and another protein, a protein and a small molecule, a nucleic acid with epigenetic modifications (e.g., DNA methylation, histone modifications), proteins with glycosylation patterns, an aptamer and a target molecule, a DNA origami structure, a protein origami structure, or other complexes.

The different categories of biological entities may relate to any biological entities present in a biological sample, such as any molecules present in the biological sample. For instance, the different binding site types of the plurality of binding site types may be configured to bind molecules in two or more categories selected from a group comprising DNA, RNA, proteins, metabolites, lipids, sugars, vitamins, hormones, and aptamers. It should also be realized that the binding sites may be specific for binding a specific molecule within a category. Alternatively, a binding site may be generic for binding any molecule of a category. It should also be realized that some binding sites may be specific and some binding sites may be generic.

This implies that the system may allow analyzing the biological entities, such as molecules, of the biological sample in multiple omics fields. Thus, information from multiple omics fields may be acquired from a single specimen of biological sample. For instance, the biological molecules from the biological sample may be analyzed in transcriptome, genome, proteome, metabolome, lipidome, glycome, and/or other omics fields.

The system may further enable the biological entities being bound at the binding sites to be identified, sequenced, and/or characterized. This may be achieved by providing a plurality of different probes and/or tags, such as fluorescent tags, to the biological entities, wherein each tag may be configured to bind to a distinct biological entity, such as a distinct molecule for identifying the molecule. In some embodiments, the tags may comprise a sequence of functional groups, such as nucleotides, configured to bind to a portion of the molecule having a complimentary sequence of functional groups. For instance, the plurality of different tags may be provided sequentially in time, such that a response from the binding site at a particular point in time could be associated with a particular tag for identifying the molecule at the binding site. Hence, the response(s) from a single binding site could be used for identifying the molecule(s) being bound at the binding site. Further, different tags may be provided sequentially for binding to different parts of a molecule. This may be used for determining presence of one or more sequences of functional groups in a molecule providing detailed information of the molecule, or even for sequencing of the molecule. For instance, a tag may correspond to one base or a plurality of bases of the molecule.

The probes or tags may comprise a first portion for binding to the biological entity that is bound at the sensor site. This first portion may for instance be a monomeric unit of a polymer (DNA, RNA, protein, etc.), a base-specific nucleotide triphosphate, a multimer nucleic acid fragment, an oligonucleotide probe, an antibody-conjugated probe, an aptamer-based probe, a protein-based probe (peptide, antibody, nanobody), an epitope-based probe, a CRISPR-based probe, or a hybrid (e.g., aptamer-antibody conjugate, peptide-nucleic acid).

The probes or tags may further comprise a second portion which may interact with illumination light such that the probe and/or tag being bound to the biological entity may be detected. For instance, the second portion may be a fluorophore providing a fluorescent response to the illumination light.

The probes or tags may be configured to releasably bind to the biological entities. Thus, a first probe or tag may bind to a molecule at a binding site at a first point in time at which response to illumination light is detected. Then, the probe may be forced to release from the biological entity and washed away before a second probe or tag is provided to bind to the molecule. Then, the second probe or tag may bind to the molecule at the binding site at a second point in time at which response to illumination light is again detected. Thus, using a sequence of probes or tags, information of the molecule bound to the binding site may be acquired.

The series of probes or tags may provide detailed information of a single biological entity. The system may detect which probes or tags in the sequence that bind to the single biological entity, forming information of a combination of multiple probes or tags that may bind to the biological entity. Alternatively, a single probe or tag may provide an identification of the biological entity. Thus, the sequence of probes or tags may allow identification of different biological entities.

A molecule may be prepared before analysis to control tags to be added one at a time to a sequence of the molecule. For instance, a fluorophore may block further nucleotides to be added to the sequence of the molecule. In this manner, a sequence of the molecule may be determined by determining one base at a time based on sequentially providing tags and removing the fluorophore after determining of the base and before providing a following tag.

In addition, a biological entity may be prepared by forming a plurality of fragments of the biological entity, which may be individually analyzed at binding sites. Information from the plurality of fragments may be used for determining sequencing of the molecule.

The biological sample may comprise a plurality of cells. Thus, the biological sample may provide an arrangement of cells in relation to each other. The biological sample may be arranged at the functionalized surface and the analysis of the biological entities may allow spatial analysis of the biological sample arranged at the functionalized surface. The arrangement of the cells may be analyzed, using the spatial analysis of the biological sample. The spatial analysis may provide analysis of different cells in the biological sample by detecting molecules from respective cells.

The biological sample may be formed by a tissue sample, which may for instance be acquired from a cultivation of cells or be extracted from a living organism, such as from a human being. However, the biological sample may alternatively comprise a sample of biological fluid extracted from a tissue sample, which may also be acquired from a living organism, such as from a human being.

The binding sites may be configured for binding single biological entities per binding site. Thus, each binding site may be configured for binding a single biological entity, e.g., by a size of the binding site allowing a single biological entity to be bound at the binding site. This may facilitate sequencing of the biological entity and may also facilitate determining information pertaining to a particular biological entity. However, each binding site may alternatively be configured for binding a plurality of biological entities.

The functionalized surface is a surface that has been modified or treated in some way to allow for specific biological entities, such as specific molecules, to be bound at the binding sites. The functionalized surface may thus be provided with surface chemistry for defining the binding sites.

For instance, the functionalized surface may be provided with a coating that provides a desired function to the functionalized surface. The functionalized surface has been modified or treated in areas corresponding to the binding sites in order to enable specific molecules to be bound at respective binding sites. Since different binding sites are configured to bind different molecules, the different binding sites are modified or treated in different manner, e.g., with different coatings.

The binding sites may each be configured to capture a particular category of molecules. Thus, the binding sites may be provided with polyadenylation (poly-A) to be configured to bind to RNA, or functional groups to bind to proteins or DNA. The binding sites may thus bind a particular category of molecules that may thereafter be identified, sequenced, and/or characterized, for instance by using a sequence of probes or tags. Each binding site may be provided with a binding element configured to capture a molecule, such as configured to capture a particular category of molecules. Different binding elements are configured to capture different molecules. Thus, the binding sites of different binding site types may be provided with different binding elements for ensuring that different molecules are bound at different binding sites.

The binding sites may also or alternatively be provided with binding elements that may provide a specificity in binding of molecules. For instance, binding elements that may be provided at the binding sites may include single-stranded DNA/RNA, which may be hybridized with complementary single-stranded DNA/RNA captured at the binding site; antibody (for proteins) or antigen (for small biomolecules) which may bind with complementary antigen or antibody, respectively; DNA aptamer; streptavidin for binding biotin or vice versa; designed DNA origami structures; protein origami structures; molecular imprinted polymers (MIP), etc.

However, the binding sites need not necessarily be provided with binding elements that may directly capture the biological entities for binding the biological entities. For instance, a binding site may be prepared for specifically receiving a binding element. Thus, the binding site may be provided with a receiver element, which may be configured to bind a particular binding element to the binding site. This implies that the binding element may be provided during or shortly prior to analysis of the biological sample for providing respective binding elements to respective binding sites. The receiver element at a binding site may thus bind a specific binding element to the binding site and the specific binding element may in turn be configured to capture and bind specific biological entities to the binding site.

The biological entities being bound at the binding sites implies that the biological entities bind to the binding site either permanently or temporarily. It should be realized that, in the embodiments described herein, the biological entities may be permanently immobilized to the binding sites, such as via covalent chemical bonding or other types of strong bonding. However, it should also be realized that the biological entities may be temporarily bound to the binding sites, such as via various types of weak bonding, such antibody-antigen recognition, DNA hybridization, electrostatic interactions, etc.

A binding strength of the biological entity to the binding site is sufficient for the biological entity to maintain a binding to the binding site at least during measurements on the biological entity. The binding site may be provided with a binding element that is configured to bind to a specific feature of a target biological entity, such as to a sequence of functional groups, e.g., amino acids or nucleotides. A chemical bond, such as a covalent bond, may be formed between a structure at the binding site and the biological entity for providing binding of the biological entity to the binding site. This provides stable binding of the biological entity to the binding site, ensuring that the biological entity is bound at the binding site during measurement. The molecules being bound at the binding sites may, at least in some embodiments, be referred to as hybridization of a probe to the molecule.

The biological entities may permanently or temporarily bind to the binding sites. The biological entities may, in embodiments, covalently bind to the binding sites and may, in other embodiments, non-covalently bind to the binding sites.

The biological entities may be configured to bind to the binding sites at certain conditions. The biological entities may further be configured to release from the binding sites at other conditions. Thus, the system may be configured to control conditions for controlling binding of biological entities to binding sites or release of biological entities to binding sites. Conditions affecting binding or release of biological entities may include buffer ionic strength, composition, temperature, or light exposure.

The conditions may be controlled for controlling selective binding of biological entities to binding sites or selective release of biological entities from binding sites. For instance, conditions may be controlled such that one or more molecules bind to binding sites at a certain point in time, while other molecule(s) do not bind to binding sites.

Although the binding sites are configured to bind biological entities, during use of the system each of the binding sites can but need not simultaneously bind biological entities. Thus, during use, biological entities may be bound at some binding sites while no biological entities are bound at other binding sites. Alternatively, during use, biological entities may be bound at each of the binding sites.

The binding sites of respective binding site types are distributed across the plurality of binding sites. Binding sites of each binding site type may thus be provided across the functionalized surface such that the system may provide spatial analysis of the biological sample with respect to each of the biological entities bound by the binding sites. This may be achieved by the binding sites of different binding site types being arranged in a regular manner within the plurality of binding sites. For instance, the binding sites of different binding site types may be arranged in a pattern which is repeated across the functionalized surface. However, it should be realized that the binding sites may alternatively be arranged in an irregular or random pattern while the binding sites still being distributed across the functionalized surface.

In case of a regular pattern of binding sites of different binding site types, closest neighbors to a binding site of a first binding site type may always be binding sites of the same binding site types, which may be different than the first binding site type. In case of an irregular pattern, closest neighbors to a binding site of the first binding site type may be different in different locations in the pattern. In some locations, two binding sites of the same binding site type may be closest neighbors.

The plurality of binding sites may be divided into a plurality of subsets. Each subset is associated with a respective light-detecting area in the array. Thus, each light-detecting area may be configured to receive light from the binding sites in the subset with which the light-detecting area is associated.

Neighboring subsets may partially overlap. Also, it should be realized that a subset may not necessarily relate to complete parts of binding sites. Rather, a border of a subset may cross a binding site such that only part of the binding site belongs to the subset. However, the subsets may advantageously be disjoint, and each subset may comprise one or more complete binding sites. This implies that the light response from a binding site may be exclusively detected by a particular light-detecting area.

The subsets may be defined by the arrangement of the light-detecting areas in relation to the plurality of binding sites. This arrangement defines which binding sites that will provide responses to the illumination pattern to respective light-detecting areas, which in turn defines the subsets.

Each light-detecting area may have an identical size. This also implies that the subsets of binding sites may have identical size.

A subset may comprise a single binding site. This implies that the response to the illumination pattern from the binding site may be provided to a unique light-detecting area, associated with the subset. Hence, light received by a light-detecting area may be directly linked to the single binding site with which the light-detecting area is associated. This may provide a simple manner of differentiating responses to the illumination pattern from different binding sites since an origin of a response may be determined based on which light-detecting area that detected the response.

In some embodiments, a light-detecting area may be associated with a part of a binding site. Thus, different light-detecting areas may be able to acquire information from different parts of a binding site. This may be used if the binding site has a relatively large size.

The illumination pattern may illuminate a single illumination area associated with a subset at a time. Thus, the light detected by the light-detecting area may be related to the illumination area that is illuminated at the point in time of detecting light.

The illumination pattern may be used for sequentially illuminating the entire subset with which the light-detecting area is associated. This may be performed for sequentially illuminating different parts of a single binding site or for sequentially illuminating different parts of a plurality of binding sites.

The illumination pattern may comprise spatial frequency content. The frequency content of the illumination pattern may comprise higher frequencies than a spatial frequency content of the arrangement of binding sites at the functionalized surface. This implies that information from different binding sites may be distinguished based on light detected in response to multiple illumination patterns.

Having subsets comprising a single binding site means that a spatial resolution provided by the system is limited by a pitch of the light-detecting areas in the array. Hence, the pitch may need to be small in order for the system to be able to provide a high resolution.

According to embodiments, each subset may comprise more than one binding site. For instance, each subset may comprise one binding site per binding site type, or a plurality of binding sites per binding site type. The system is still configured to enable differentiating responses to the illumination pattern from different binding sites. This may be achieved in several ways.

The illumination pattern may be configured to selectively illuminate one binding site of each subset at a time, such that the response detected by a light-detecting area may be associated with a specific binding site based on the time point at which the response is detected.

The illumination pattern may alternatively be configured to illuminate more than one binding site at a time. According to some embodiments, the illumination pattern may be configured to, within a subset associated with a single light-detecting area, only illuminate one binding site per binding site type at a time. Binding sites of different binding site types may be activated (provided with tags for inducing a response to the illumination pattern) at different points in time. Thus, the response detected by a light-detecting area may be associated with a binding site of a specific binding site type within an illuminated area based on the time point at which the response is detected.

The illumination pattern may be provided by structured illumination. The structured illumination may allow selectively illuminating one area or multiple disjoint areas of the functionalized surface at a time. This may be a suitable manner of selectively illuminating one or more binding sites. The illumination pattern may further be controlled for providing sequential illumination of different areas of the functionalized surface such that all of the binding sites may be illuminated.

A sequential illumination may be provided by a sequence of illumination patterns. Different illumination patterns in the sequence may have same frequency content, with the illumination pattern being spatially translated.

However, it should be realized that different illumination patterns in a sequence of illuminations may have different frequency content. The illumination patterns in a sequence of illuminations may be fully uncorrelated.

In addition, the illumination pattern may define a deterministic pattern of light at the functionalized surface. However, the illumination pattern may alternatively define a non-deterministic pattern of light at the functionalized surface.

According to some embodiments, the illumination pattern may be configured to illuminate more than one binding site of a same binding site type at a time. For instance, an illumination area in the illumination pattern may extend over more than one binding site of the same binding site type. The system may further be configured to provide sequential illumination of different illumination patterns such that responses associated with each of the binding sites may be extracted from the light detected by the array of light-detecting areas. The sequential illumination patterns may in such case need to have an overlap of areas of the functionalized surface being illuminated for allowing the responses associated with each of the binding sites to be extracted.

The illumination pattern may be known. Thus, a processor for processing the light detected by the array of light-detecting areas may have knowledge of the illumination pattern. This may be used for determining the binding sites from which light is detected in order to determine information of the biological entities and/or for reconstructing a visual image representation of the biological entities arranged at the functionalized surface.

However, the illumination pattern need not necessarily be known. Rather, processing of the light detected by the array of light-detecting areas may be performed based on blind processing (i.e., without knowledge of the illumination patterns), such as using blind deconvolution. The blind processing may be able to determine accurate reconstruction of an image of the biological entities arranged at the functionalized surface by the (unknown) illumination patterns in sum being configured to provide a uniform illumination of the field of view.

According to some embodiments, the information acquired by a light-detecting area may not allow determining an exact location of each binding site from which information is acquired within a subset. However, in some embodiments, it may not be necessary to be able to reconstruct an image of the biological entities at exact locations of the binding sites. Rather, the information acquired relating to the biological entities bound to the binding sites may be sufficient.

Further, the system may be configured to acquire information in multiple cycles from the same binding site, e.g., for determining multiple characteristics of the biological entities bound to the binding sites. The information acquired by the light-detecting area from the multiple cycles may be associated with each other, even though the locations of the binding sites from which responses are received may not be exactly determined. Thus, the information acquired by the light-detecting area in multiple cycles may be used for forming combined information from multiple cycles relating to the same biological entity or to the biological entities bound at the same binding site. This may for instance be used in sequencing of biological entities, wherein a sequence of the biological entity may be determined without the system being able to exactly reconstruct a location at which the biological entity is bound. This may also or alternatively be used for determining a distinctive pattern within a sequence of a biological entity that enables unique identification of the biological entity.

For instance, the system may be configured to provide identical sequences of illumination pattern in each cycle of acquiring information from the functionalized surface. This may facilitate associating information acquired in different cycles to the same biological entity and/or the same binding site.

The system may alternatively be configured to provide different illumination patterns in different cycles. Information from different binding sites may be distinguished based on light detected in response to multiple illumination patterns provided at different cycles.

Structured illumination may be used for providing a high resolution of selecting area(s) of the functionalized surface that are illuminated. Also, this implies that the imaging of the functionalized surface provided by a sequence of detected responses may be further processed for spatially relating sequential responses to each other, such as for providing sequencing of a biological entity, or through image reconstruction for achieving a reconstructed image of the binding sites.

It should be realized that the illumination pattern may provide a response from a particular location at the functionalized surface corresponding to a biological entity (or a tag bound to the biological entity) that absorbs light. The response may thus give rise to a point spread function of light emitted from the biological entity. The light-detecting areas may advantageously be arranged in relation to the functionalized surface such that the point spread function is exclusively provided to a single light-detecting area.

The system is configured to provide an illumination pattern with multiple spatial frequencies. This may be achieved by the illumination pattern being non-uniform and providing structured illumination. The system is further configured to provide the array of light-detecting areas with a pitch of the light-detecting areas within the array. The pitch defines a spatial frequency of read-out of signals from the functionalized surface. The resolution of imaging provided by the system may be limited by a highest spatial frequency in the system. This spatial frequency may be either a spatial frequency of the illumination pattern or the pitch of the array of light-detecting areas.

Further, it should be realized that the illumination pattern may be configured to define discrete illumination areas, each selectively illuminating one or more binding sites. The arrangement of the discrete illumination areas of the illumination pattern may define spatial frequencies of the illumination pattern. However, within an illumination area, intensity of illumination light may vary. This may also contribute to the spatial frequency of the illumination pattern.

It should be further realized that data acquired by the light-detecting areas may be processed using an image reconstruction algorithm that can reproduce a representation of the biological sample from a series of detections by the array of light-detecting areas taken with different illumination patterns (e.g., a series formed by scanning a lattice of illumination spots, or a series formed by different speckle patterns).

It should be realized that the illumination system and the array of light-detecting areas being configured to provide imaging of the functionalized surface does not necessarily mean that the system is configured to acquire an optical image of the functionalized surface. Rather, the illumination system and the array of light-detecting areas is configured to generate a representation of properties of biological entities bound at binding sites of the functionalized surface, wherein a relation between spatial location and properties is determined. This may allow forming a visual image representing one or more properties of the sample, by direct representation of information from different binding sites and/or by processing acquired data through an image reconstruction algorithm. It should also be realized that the system need not necessarily form an actual visual image of the one or more properties of the sample. Rather, the information may be provided, e.g., as a matrix of values relating spatial locations to properties.

The illumination system being configured to selectively illuminate one or more binding sites at a time implies that the illumination system provides illumination that selectively illuminates a portion of the functionalized surface. Thus, the illumination system does not illuminate the entire functionalized surface simultaneously but rather only a part of the functionalized surface is illuminated at a time. The illumination system is further configured to sequentially change the illumination pattern in order for all of the plurality of binding sites to be illuminated. Thus, the illumination system may comprise a controller for controlling and changing the illumination pattern.

It should be realized that the light detected by the light-detecting areas is separately read out for each illumination in a sequence of illumination patterns. Thus, a read-out from the array of light-detecting areas may be performed for each illumination pattern in a sequence. Between consecutive illuminations of different illumination patterns, a reset of the light-detecting areas may be performed.

The illumination system may be arranged in any suitable manner for forming the illumination pattern. For instance, the illumination system may comprise one or more optical components that act on illumination light for forming the illumination pattern on the functionalized surface.

Also, the illumination system may be configured to form the illumination pattern at the functionalized surface from any direction in relation to the functionalized surface. Thus, the illumination system may be configured to provide the illumination pattern from above, i.e., from a medium interfacing the functionalized surface. However, the illumination system may alternatively be configured to provide the illumination pattern from below, i.e., illumination light passes through or in a layer forming the functionalized surface.

The illumination system may comprise a light source for generating illumination light used for forming the illumination pattern. However, it should be realized that the illumination system may be configured to receive illumination light from an external light source and the illumination system may further be configured to form and control the illumination pattern based on the received illumination light.

The illumination light may be generated by a light source, typically a laser light source. The illumination light may have a sufficiently large spatial coherence length for forming the illumination pattern by interference of light. The spatial coherence length may be at least 2 times a distance across the field of view for ensuring that a well-defined illumination pattern may be formed over the entire field of view. The illumination light may provide light in a single, narrow wavelength band, such that the illumination light may be monochromatic. This may be useful for providing accurate control of the illumination pattern.

It should be realized that the illumination light may have a wavelength corresponding to visible light but is not limited to visible light. Rather, the illumination light may have a wavelength in another part of electro-magnetic spectrum, such as ultraviolet light or infrared light.

The illumination pattern may be configured to cause output of light at the binding sites. The illumination light may be configured to directly excite the biological entity for causing a response to the illumination pattern. This may for instance be achieved for a biological entity being a fluorescent protein, such as green fluorescent protein. However, the illumination light may alternatively be configured to excite a tag that is bound to the biological entity at the binding site. Thus, light need not be output by the biological entity itself but may instead be output by a tag bound to the biological entity to provide output light associated with the biological entity.

The illumination light may be configured to provide excitation light for inducing photoluminescence. Thus, the output light may provide a response to the illumination pattern in form of phosphorescence or fluorescence. However, it should be realized that the illumination light may alternatively be scattered, such that the output light associated with the biological entities is scattered light. Thus, the output light may provide a response to the illumination pattern in form of scattered light, such as Rayleigh scattering or Raman scattering of light.

It should be further realized that illumination may be performed at multiple wavelength bands. Different wavelength bands may be used for inducing responses from different tags. For instance, different tags may be provided with different fluorophores that may be excited by different wavelengths of illumination light. Thus, illumination may be sequentially applied at different wavelengths for inducing responses from different tags and hence sequentially acquiring information associated with a plurality of tags. This implies that a plurality of tags may be bound to biological entities at the same time and selection of wavelength of illumination light may be used for selectively inducing output of light from one type of tag at a time. This enables distinguishing responses from a plurality of tags that are bound to biological entities at the same time.

The array of light-detecting areas may be arranged in relation to the functionalized surface for receiving the output light forming the response to the illumination pattern. The array of light-detecting areas may for instance be arranged with the light-detecting areas extending in parallel with the functionalized surface. This is a suitable manner for ensuring that the light-detecting areas are configured to receive the output light. Further, such arrangement of the light-detecting areas in relation to the functionalized surface may ensure that the light-detecting areas are associated with a respective part of the functionalized surface (a subset of binding sites) to receive light from that part of the functionalized surface.

A light-detecting area is associated with a subset of binding sites. This implies that the light-detecting area may receive output light associated with biological entities bound at the binding sites in the subset. The subset may comprise a single binding site. This implies that each light-detecting area is configured to detect light from a unique binding site. However, according to an alternative, each light-detecting area may be configured to detect light from a plurality of binding sites of the subset.

The array of light-detecting areas may be integrated with the functionalized surface providing a fixed relation between the light-detecting areas and the functionalized surface. This may ensure that a relation between the array of light-detecting areas and the functionalized surface is maintained throughout a sequence of measurements. This further implies that image reconstruction may accurately be performed for forming an image of the biological sample and/or that sequencing of the biological entities bound at the binding sites may be accurately performed.

It should be further realized that information detected by the array of light-detecting areas may provide a representation of the plurality of biological entities, without necessarily a visual image of the biological entities being formed. Data detected by the array of light-detecting areas in one or more sequential illuminations of the plurality of biological entities may be processed in various manners. For instance, data processing may be performed to generate a multi-dimensional representation of the plurality of biological entities, which is not necessarily provided in form of a visual image. The multi-dimensional representation may for instance provide information of a sequence, molecular identity, morphology, or density of a biological entity based on acquired information, e.g., using a sequence of tags being provided to the plurality of biological entities.

The array of light-detecting areas may be configured in any manner for detecting light incident on the light-detecting areas. For instance, the light-detecting areas may be configured to convert an intensity of incident light to an electrical signal representing the intensity. The light-detecting areas may for instance be implemented as a complementary metal-oxide-semiconductor (CMOS sensor), a charge-coupled device, photodiodes, or photo-multiplier tubes. The array of light-detecting areas may be formed by an event camera, which may also be referred to as a neuromorphic camera.

Light detected by a light-detecting area in the array of light-detecting areas may be individually read out. Thus, the light-detecting areas in the array may be separate areas. Each light-detecting area may be a continuous area for detecting light incident on the area.

According to an embodiment, each subset comprises a plurality of binding sites of each binding site type.

Thus, each subset may comprise more than one binding site per binding site type. Each binding site type may be represented in the subset and for each binding site type, there may be more than one binding site.

This implies that the illumination pattern may be used for differentiating between responses from different binding sites within the subset. Responses from the plurality of binding sites within the subset may thus be provided to the same light-detecting area which is associated with the subset.

Hence, the system may utilize controlling of the illumination pattern for differentiating between responses from different binding sites. This implies that a size of each light-detecting area need not be very small in order to provide a high resolution of acquiring information relating to the biological entities.

In addition, thanks to the illumination enabling differentiating between responses from different binding sites, the binding sites may be provided at a very high resolution. For instance, a high resolution patterning of the binding sites may be provided. A subset comprising a plurality of binding sites per binding site type implies that the resolution of patterning of binding sites within the same binding site type may be high and not limited by the size of the light-detecting area. This, however, implies that accurate control of the illumination may be needed in order to provide a higher spatial frequency by the illumination compared to the spatial frequency of the arrangement of the binding sites, to enable distinguishing responses from two binding sites of the same binding site type within the same subset.

Thus, a high density of binding sites may be provided without requiring a small pitch of the light-detecting areas in the array. This may ensure that information may be efficiently acquired by the system and that a high throughput for analysis of biological entities may be provided. This also implies that the system may provide a cost efficient manner of acquiring information for analysis of biological entities.

According to an embodiment, the illumination system is configured to form the illumination pattern comprising one or more illumination areas, wherein each illumination area is illuminating a single binding site of a same binding site type in the subset at a time.

This implies that the illumination area may be configured to selectively illuminate a particular binding site at a particular point in time. Hence, the response detected by the light-detecting area associated with the subset may be determined to originate from the illuminated binding site.

Different binding site types are configured to bind different categories of biological entities. The system may further be configured to sequentially provide different tags that will bind to different categories of biological entities, which may ensure that responses from different binding site types may not be provided simultaneously, even if the binding sites of different binding site types would be simultaneously illuminated. Hence, in order to enable determination of which binding site a detected response originates from, it may be sufficient that the illumination area has an extension so as to only illuminate a single binding site of the same binding site type.

According to another embodiment, the illumination system is configured to form the illumination pattern comprising one or more illumination areas, wherein each illumination area is illuminating a plurality of binding sites in the subset. The illumination area may be illuminating a plurality of binding sites of the same binding site types.

The system may further be configured to selectively provide a response to the illumination by biological entities bound to a single binding site type. The system may be configured to sequentially provide tags for binding to the biological entities. Different tags may be provided for binding to biological entities that are bound to different binding site types. Thus, at a particular point in time, tags may only be bound to biological entities that are bound to binding sites of a specific binding site type. Hence, responses to the illumination may only be provided from biological entities bound at binding sites of a single binding site type. The tags being sequentially provided may ensure that responses from biological entities bound to binding sites of different binding site types are provided sequentially in time.

Responses from biological entities bound to different binding sites of the same binding site type may be distinguished by an illumination area only illuminating one binding site of the same binding site type within the same subset. Thus, the light-detecting area may only receive light from a biological entity being bound to a single binding site.

Alternatively, responses from biological entities bound to different binding sites of the same binding site type may be distinguished by the sequential illumination providing a higher spatial frequency than the arrangement of the binding sites at the functionalized surface. This implies that a contribution from a single binding site within responses detected from a plurality of illumination patterns may be determined.

According to yet another embodiment, the illumination system is configured to form the illumination pattern with each illumination area illuminating a part of a single binding site at a time. This may facilitate acquiring information of parts of a biological entity bound at the binding site.

According to an embodiment, the illumination system is configured to form the illumination pattern comprising more than one illumination area, wherein each illumination area is associated with one or more unique subsets.

Thus, the system may be configured to simultaneously illuminate different parts of the functionalized surface. Thanks to each illumination area being associated with a unique subset of the plurality of binding sites, a single illumination area per subset may be provided at a time.

Further, each light-detecting area is associated with a unique subset. Thus, each light-detecting area may detect a response from a single illumination area. This enables easily differentiating between detected responses from different illumination areas, since the responses from different illumination areas are detected by different light-detecting areas.

It should also be realized that the illumination pattern may be configured to provide illumination to all subsets at the same time. Thus, each light-detecting area may be configured to receive a response based on a respective illumination area being configured to illuminate the subset of the binding sites with which the light-detecting area is associated.

However, according to other embodiments, the illumination area(s) do not illuminate all of the subsets at the same time. For instance, the illumination pattern may comprise one illumination area per multiple subsets. During sequential illumination, location of the illumination area may be changed so as to provide illumination of the binding sites of the multiple subsets.

The illumination areas may be arranged in relation to each other at the functionalized surface such that point spread functions of output light induced by neighboring illumination areas do not overlap at a plane of the array of light-detecting areas. Thus, the illumination areas may be sufficiently spaced apart for avoiding overlap of point spread functions.

According to an embodiment, the illumination system comprises an illumination waveguide integrated with the functionalized surface and configured to propagate one or more light waves for forming the illumination pattern based on the one or more light waves. The illumination system may further comprise a controller which is configured to control the one or more light waves propagating in the illumination waveguide for sequentially illuminating different binding sites.

The illumination pattern may be controlled by controlling propagation of light waves or a relation between light waves in the illumination waveguide. Thus, the illumination system may allow changing the illumination pattern for illuminating an entire field of view, without requiring any mechanical movements. This implies that the system is robust.

In addition, the biological entities may be illuminated based on the illumination pattern in the illumination waveguide being directly received at the functionalized surface. Thus, illumination light need not travel between the illumination waveguide and the functionalized surface. This implies that the system provides an accurate control of the illumination areas being illuminated at the functionalized surface without a need for precise alignments between an illumination system and a surface at which biological entities to be analyzed are provided.

The illumination waveguide may be provided such that illumination light need not pass through parts of a sample before reaching biological entities bound at the binding sites. In addition, the illumination light need not pass through other layers of material between the illumination waveguide and the biological entities. This may ensure that noise or interfering signals caused by interaction of illumination light with other matter not of interest may be avoided.

The illumination waveguide may be any structure providing guiding of light. The illumination waveguide may be configured to confine light in a cross-section perpendicular to an extension of the waveguide. The illumination waveguide may thus be configured to restrict light to follow a path defined by the extension of the illumination waveguide, whereby transmission of light with low loss may be provided. The illumination waveguide may for instance guide light by light being reflected on inner walls of the illumination waveguide by total internal reflection or by a reflective coating being provided on the walls of the illumination waveguide. The illumination waveguide is configured to propagate the one or more light waves along the path of the illumination waveguide.

The illumination waveguide may have a relatively large extension in the plane in which the illumination waveguide is arranged. This implies that the illumination waveguide may provide light to a large area of the binding sites.

The illumination waveguide may be formed by any suitable core material surrounded by a suitable cladding material. Thus, the light may be propagated in the core material and may be reflected by total internal reflection in an interface between the core material and the cladding material. Thus, the core material may have a higher refractive index than the cladding material. The core material may be any suitable material for propagating the light with low loss, i.e., having low absorption in the wavelength range of light. For instance, the illumination waveguide may be formed by a core material of silicon or silicon nitride surrounded by a cladding material of silicon dioxide. However, it should be realized that other materials may be used.

The functionalized surface may present a surface for receiving biological entities, e.g., biological entities of a sample. The illumination waveguide may be arranged in relation to the functionalized surface in an opposite direction to which the functionalized surface is facing for receiving the biological entities. Thus, the illumination waveguide may be considered to be arranged below the functionalized surface for providing illumination of the functionalized surface.

The functionalized surface may be a surface of a layer that is part of the illumination waveguide, such as the functionalized surface forming a surface of the cladding material or a surface of the core material. The functionalized surface may face away from the illumination waveguide.

The functionalized surface may thus be arranged in relation to the illumination waveguide to directly receive light from the illumination waveguide. Alternatively, one or more layers of material may be arranged between the illumination waveguide and the functionalized surface.

Thanks to the illumination waveguide being integrated with the functionalized surface, the system may be very compact.

Surface chemistry of the functionalized surface forming the binding sites may be realized in close proximity of the illumination waveguide that creates the illumination pattern.

The functionalization of the surface may advantageously occur on a flat planar surface in the close proximity of the waveguide. This may ensure uniform dielectric space in which the illumination pattern is created, without distortions being formed originating from heterogeneous dielectric space.

A process for development of the functionalized surface may require a non-flat surface to achieve high confinement and low crosstalk between binding sites. The functionalization process may be adapted to create temporary sacrificial structures for isolation to realize high density patterning of binding sites. Sacrificial nature of the patterning may result in a flat planar surface after the surface functionalization is done and sacrificial patterns are removed. This facilitates a known illumination pattern without distortions that ensures the binding sites are illuminated as expected.

The illumination waveguide is configured such that light may be received at the functionalized surface from the illumination waveguide.

The one or more light waves propagating in the illumination waveguide may interfere. Thus, the illumination pattern may be formed based on interference of the one or more light waves.

A single light wave may interfere with itself, e.g., by the light wave being reflected at an edge of the illumination waveguide and hence traveling back in the illumination waveguide to interfere with itself. Two or more light waves being introduced in the illumination waveguide, e.g., at different input locations, may also interfere within the illumination waveguide.

Interference of the light waves may cause an interference pattern of constructive and destructive interference of the one or more light waves. Thus, the illumination pattern may be based on interference such that an intensity of light of the illumination pattern in a particular area is dependent on whether constructive or destructive interference of the one or more light waves occurs in the particular area. The illumination area of the illumination pattern may thus be defined by a region in the illumination waveguide in which constructive interference of the one or more light waves is provided.

It should be realized that the constructive interference may be defined at a surface of the illumination waveguide in which total internal reflection occurs. This constructive interference may in turn result in an area of the functionalized surface receiving light for defining the illumination area of the illumination pattern at the functionalized surface.

The illumination system may be configured to control interference of the one or more light waves for controlling the illumination pattern. This may be used for controlling sequential illumination of different parts of the functionalized surface.

For instance, the illumination system may be configured to control the illumination pattern by controlling the light waves that are input to the illumination waveguide and/or by controlling reflection of one or more light waves at an edge of the illumination waveguide. For instance, a phase and/or a wavelength of the light waves may be controlled or a location at which the light waves are provided into the illumination waveguide may be controlled. According to yet another alternative, a propagation property of the illumination waveguide may be locally controlled for changing relations between light waves or different parts of light waves propagating in the illumination waveguide.

The controller is configured to control a sequence in time of forming different illumination patterns. Thus, a sequence in time of illumination patterns is formed.

The controller may ensure that the illumination pattern is properly controlled for allowing the illumination pattern to be sequentially changed. The controller may for instance be configured to control the one or more light waves such that a location of an illumination area is sequentially moved between different illumination times across an area of the functionalized surface. The illumination area may be moved in steps for scanning the functionalized surface.

However, it should be realized that the illumination pattern need not be changed in a sequence in time such that the illumination area sequentially moves across the area of the functionalized surface. Rather, the illumination area may be moved in any manner between different illumination patterns in a sequence in time. For instance, a difference in position of the illumination area between two consecutive illumination patterns need not be the same for different pairs of consecutive illumination patterns.

The controller may for instance be configured to control a phase of light propagating in one or more input waveguides. Each input waveguide may provide a light wave into the illumination waveguide. By controlling the phase of light propagating in the input waveguides, a relation between the light waves input to the illumination waveguide may be changed which may also change the illumination pattern formed in the illumination waveguide.

Each input waveguide may be associated with a phase shifter for controlling the phase of the light wave propagating in the input waveguide. The controller may thus be configured to provide control signals to the phase shifters for controlling relative phase shifts induced in different input waveguides. This may be a simple manner of controlling the illumination pattern.

The phase shifter may for instance be a thermal phase shifter providing control of phase shift by controlling a temperature of the input waveguide. The phase shifter may alternatively be an electro-optic phase shifter or an acousto-optic phase shifter for controlling phase shifts of light waves propagating in the input waveguide.

Alternatively or additionally, the controller may be configured to control coupling conditions between the input waveguide and the illumination waveguide, which may control relative phase shifts between light waves. The coupling conditions may be controlled for controlling an angle of direction of a light wave being introduced into the illumination waveguide or a location at which a light wave is coupled into the illumination waveguide. This may be controlled, for instance, by the input waveguide being arranged to extend along a portion of the illumination waveguide, such that coupling conditions may control how a light wave is coupled from the input waveguide into the illumination waveguide.

According to an embodiment, the controller is configured to control the one or more light waves propagating in the illumination waveguide for sequentially forming different illumination patterns comprising illumination areas configured to illuminate each of the binding sites, wherein the illumination areas of the different illumination patterns in combination cover a combined area of all of the binding sites.

Thus, the controller may ensure that sequential illumination is used for illuminating a combined area of the binding sites at the functionalized surface. This may ensure that information from all of the binding sites may be acquired.

The controller may be configured to control the illumination patterns such that illumination area(s) are moved in a structured manner between different illuminations in a sequence for scanning the functionalized surface. However, the sequential illumination patterns may be changed in different manners for illuminating the combined area of all of the binding sites.

The illumination areas of different illumination patterns may be disjoint. However, according to an alternative, illumination areas of different illumination patterns may overlap. This may be used for determining information from different parts of the functionalized surface and from different parts of binding sites for providing high resolution of information detected from the functionalized surface.

It should further be realized that a sample may be arranged in a predefined pattern in relation to the functionalized surface. Alternatively, the system may be configured to determine a pattern in which a sample is placed on the functionalized surface. This may imply that information need only be acquired from certain parts of the binding sites at the functionalized surface and/or information may only need to be determined for certain parts of the binding sites.

Thus, the controller may be configured to control the one or more light waves propagating in the illumination waveguide for sequentially forming different illumination patterns comprising illumination areas configured to illuminate parts of the binding sites in which biological entities are present. This implies that the illumination may be limited only to interesting parts of the functionalized surface.

In addition, the detected responses may be processed so as to only determine a representation of information in locations at the functionalized surface where biological entities are arranged. This may ensure that processing of information is efficient.

According to an embodiment, the array of light-detecting areas is integrated with the illumination waveguide.

This may ensure that a relation between the array of light-detecting areas and the functionalized surface is well-known. In addition, the relation between the array of light-detecting areas and the functionalized surface is maintained throughout a sequence of measurements. This further implies that image reconstruction may accurately be performed for forming an image of a biological sample and/or that sequencing of the biological entities bound at the binding sites may be accurately performed.

The array of light-detecting areas being integrated with the illumination waveguide further implies that the system may be very compact.

The array of light-detecting areas may for instance be configured to extend in a plane parallel to a plane of the illumination waveguide in which propagation of the light waves is provided and parallel to the functionalized surface. This may provide a well-defined relation between illumination areas and the light-detecting areas.

The illumination waveguide may be arranged between the array of light-detecting areas and the functionalized surface. This may be a suitable manner of arranging the illumination waveguide and the array of light-detecting areas in relation to the functionalized surface for providing both illumination of the functionalized surface and detection of responses from the functionalized surface. It should be realized that the illumination waveguide may be arranged between the array of light-detecting areas and the functionalized surface also in embodiments where the array of light-detecting areas is not integrated with the illumination waveguide.

According to an embodiment, the system is configured to provide time-gated detection of light by the array of light-detecting areas and/or the system comprises a filter for selectively passing wavelengths to the array of light-detecting areas.

The system may be configured to differentiate output from different tags. This may be achieved by activating tags at different points in time, e.g., based on the tags being excited by different wavelengths of illumination light. However, the system may also be configured to differentiate output from different tags, even if the tags provide output at the same time.

For instance, different tags may be provided with different fluorophores such that different wavelengths of light may be provided by different tags. The system may comprise a filter that is configured to selectively pass different wavelengths. This may be used for ensuring that different sub-areas of a light-detecting area receive different wavelengths of light, e.g., based on a patterned filter.

In addition or alternatively to providing different wavelengths of light, different tags may provide different lifetime of output of light after being excited by illumination light. This may be used for differentiating output from different tags. The system may thus be configured to provide time-gated detection of light by the array of light-detecting areas for enabling differentiating output from different tags.

The time-gated detection of light may be associated with lifetime of output of light associated with the biological entity. The system may be configured to read out detected light using two or more time gates, allowing determination of a slope of intensity of detected light. Thus, the time-gated detection may provide a differentiation in lifetime of the output light, such that the tag providing the output of light may be identified.

According to an embodiment, a size of each of the binding sites is smaller than 1000 nm, such as in a range of 1 - 750 nm, such as in a range of 5 - 500 nm, and an extension of an illumination area in the illumination pattern is smaller than 10 µm, such as in a range of 100 nm - 10 µm.

This implies that the system is able to acquire information relating to the plurality of biological entities with high resolution.

Thanks to using small illumination areas, the system provides high resolution. The small illumination area may be smaller than a size of a light-detecting area. This implies that resolution of acquisition of information is not limited by the size of the light-detecting area. In addition, the system need not use a bulky imaging system, such as an imaging system including one or more lenses, for providing a high resolution. Rather, a high resolution may be provided thanks to controlling a size of the illumination area in the illumination pattern.

The binding sites may also aid in providing resolution of acquiring of information by using a small size of binding sites. A binding site may be configured to capture a specific biological entity. Thus, a location of the biological entity in a sample may be determined with high accuracy based on at which binding site the biological entity was bound.

The extension of the illumination area may be an extension of the illumination area in a direction parallel to the functionalized surface. The extension may correspond to a distance between opposite edges of the illumination area. Thus, for a circle-shaped illumination area, the extension may be a diameter of the illumination area. For a square-shaped illumination area, the extension may be a size of a side of the illumination area.

It should be realized that the illumination areas may have any shape. The illumination areas may form spots, such as substantially circular or elliptical spots, such that a largest dimension of the spot (e.g., a diameter) may have a size in the range of 100 nm - 10 µm. Alternatively, the illumination areas may have more irregular shapes.

Also, the illumination areas may form lines or elongate shapes, which may have a large extension in a first direction. However, such elongate illumination areas may be smaller in a second direction, perpendicular to the first direction, so that the illumination areas in the second direction have a dimension which is relatively small, such as in the range of 100 nm - 10 µm.

The illumination area may provide a resolution of acquiring information of the biological entities bound at the binding sites. The illumination area may be small in order to provide a high resolution. Thus, in some embodiments, the illumination area may have an extension that is smaller than 1000 nm, such as in a range of 100 nm - 1000 nm, or in a range of 100 nm - 500 nm.

However, it should be realized that it may still be beneficial to provide resolution via illumination areas for more coarse resolutions, such that a resolution of up to 10 µm may be relevant using the technology of controlling the illumination pattern.

According to an embodiment, the illumination system is configured to illuminate the biological entities bound at binding sites for inducing the output light associated with the biological entities, such as fluorescence light, wherein the system is configured to differentiate output light associated with different biological entities bound by different binding sites by at least one of: a point in time at which the output light is induced, an emission wavelength of the output light, an intensity of the output light, or a lifetime of the output light.

According to another embodiment, the illumination system is configured to illuminate the biological entities bound at binding sites for inducing the output light associated with the biological entities, such as fluorescence light, wherein the system is configured to differentiate output light associated with different parts of biological entities by at least one of: a point in time at which the output light is induced, an emission wavelength of the output light, an intensity of the output light, or a lifetime of the output light.

The system may know the illumination pattern being provided. Thus, a detected response at a particular point in time may be directly associated with the area of the functionalized surface being illuminated at the particular point in time. This may be used for determining information for identifying the biological entity at a particular binding site and/or may be used for determining detailed information of the biological entity, such as for sequencing of a molecule.

The output light associated with a biological entity may be dependent on characteristics of the biological entity and/or characteristics of a tag binding to the biological entity. For instance, different tags configured to emit light at different emission wavelengths may be provided. Thus, detection of an emission wavelength of the output light may be used for determining a tag that provided the response to the illumination light. This may further be used for determining information relating to the biological entity.

Different tags may alternatively or additionally be configured to emit light with different intensity of the response and/or with different lifetime of the response. Thus, intensity of the output light and/or lifetime of the output light may additionally or alternatively be used for determining a tag that provided the response to the illumination light. This may further be used for determining information relating to the biological entity.

According to an embodiment, the illumination system is configured to form the illumination pattern for selectively illuminating the binding site by an evanescent field and configured to induce output of fluorescence light.

The illumination system may further be configured to induce output of fluorescence light based on the evanescent field being absorbed by biological entities and/or tags bound to biological entities at the binding site.

The illumination system may comprise an illumination waveguide as discussed above. The illumination waveguide may propagate light using total internal reflection in the illumination waveguide. The light propagating in the illumination waveguide may comprise an evanescent field at surfaces in which total internal reflection occurs. The evanescent field extends a small distance out of the illumination waveguide.

Thus, using illumination by an evanescent field implies that a small portion of light escaping a surface in which total internal reflection occurs may be used for illumination and intensity of the light will very quickly decrease with distance from the surface.

The use of a light wave propagating through total internal reflection in the illumination waveguide allows the biological entities to be illuminated using an evanescent field of the propagated light wave outside the illumination waveguide. Since an intensity of the evanescent field quickly decreases with distance from the illumination waveguide, this implies that only biological entities or portions of biological entities arranged very close (in the order of 50-100 nm) to the illumination waveguide will be illuminated by the evanescent field. Hence, the use of the illumination waveguide providing illumination using the evanescent field sets a vertical resolution above the illumination waveguide, such that a response to illumination light will only be triggered at the functionalized surface or very close to the functionalized surface.

The use of the evanescent field for inducing output of light also implies that a response may be detected with a high signal-to-noise ratio. For instance, in comparison with illumination being provided through a sample arranged at a functionalized surface, such illumination may give rise to light being output not only by biological entities arranged at the functionalized surface so as to cause a background noise at the light-detecting areas.

The system may be configured to control intensity of illumination light between sequential illuminations. Thus, different intensities may be used in different illumination patterns during a sequence of illuminations.

This may be particularly useful when the system is used for detecting information from a single biological entity using multiple sequential tags. For instance, if the system is used for sequencing of a biological entity by adding tags in a sequence to the biological entity, the system may need to detect information in a large number of sequential steps. Further, if a long sequence is to be detected, a vertical position of the tag may increase in the sequence. Thus, a relatively high intensity of illumination light may be needed towards an end of the sequence in order to allow detection of information provided by tags that are bound to the biological entity at the binding site in a position relatively far away from the surface of the illumination waveguide.

However, if a high intensity is provided for all illuminations in the sequence of illuminations, photon damage may occur to parts added to the biological entity in beginning of the sequence. Hence, using a varying intensity of the illumination light such that different intensities are used for different parts of a sequence of illuminations, the system may allow detecting information from tags provided relatively far away from the surface of the illumination waveguide while avoiding photon damage.

According to an embodiment, the system further comprises a filter arranged between the functionalized surface and the array of light-detecting areas for rejecting an excitation wavelength and passing a fluorescence wavelength of output light.

As mentioned above, the system may comprise an illumination waveguide. The filter may then be arranged between the illumination waveguide and the array of light-detecting areas for rejecting an excitation wavelength propagating through the illumination waveguide.

The illumination waveguide may be configured to propagate light using total internal reflection. Thus, no or small amount of light propagating in the illumination waveguide should be able to leave the illumination waveguide towards the array of light-detecting areas.

Further, the illumination light, e.g., illumination light propagating in the illumination waveguide, may have an excitation wavelength and may be configured to induce output of light at another wavelength. For instance, if the illumination light induces fluorescence, the output light associated with the biological entity, may have a fluorescence wavelength that is longer than the excitation wavelength. Thus, using a filter, fluorescence light may be selectively passed towards the light-detecting areas while illumination light may be prevented from passing the filter.

The filter may be arranged in a layer between the illumination waveguide and the array of light-detecting areas. For instance, the filter may be arranged between the illumination waveguide and the array of light-detecting areas in an integrated device where the array of light-detecting areas is integrated with the illumination waveguide. The filter may be configured to filter light based on wavelength, which is a simple manner of selectively passing fluorescence light.

The filter may for instance be an absorption filter or an interference filter. The filter may be configured to pass a specific wavelength corresponding to the fluorescence wavelength forming a band-pass filter. The filter may alternatively be configured to prevent a specific wavelength corresponding to the excitation wavelength from passing the filter forming a band-stop filter. As yet another alternative, the filter may be configured to pass wavelengths longer than the excitation wavelength, such that the filter may be a high-pass filter with a cut-off wavelength between the excitation wavelength and the fluorescence wavelength.

According to an embodiment, the system further comprises an optical modulator arranged between the functionalized surface and the array of light-detecting areas for shaping output light towards the array of light-detecting areas. If the system comprises the illumination waveguide, the optical modulator may be arranged between the illumination waveguide and the array of light-detecting areas.

The output light in response to illumination may be give rise to a point spread function (PSF) of light emitted from the biological entity. The optical modulator may be configured to shape the PSF, e.g., by shaping the point spread function locally. Thus, the PSF may be spatially variant, which may provide additional information for processing detected light. This may for instance be used for increasing resolution or read-out density for acquiring information of the biological entities.

The optical modulator may alternatively be configured to generate different PSFs at different wavelengths of light. This may be used for differentiating between different wavelengths of output light from the biological entities.

The optical modulator may alternatively be configured to generate different responses for different angles of incident light at different wavelengths. This may also be used for differentiating between output light at different wavelengths from the biological entities.

The optical modulator may be arranged between the illumination waveguide and the array of light-detecting areas. For instance, the optical modulator may be arranged between the illumination waveguide and the array of light-detecting areas in an integrated device where the array of light-detecting areas is integrated with the illumination waveguide. The optical modulator may be arranged at a distance from the illumination waveguide so as to avoid scattering of the illumination light. For instance, a spacer layer may be arranged between the illumination waveguide and the optical modulator.

The optical modulator may for instance be provided by a patterned optical layer for providing modulation. The patterned optical layer may for instance be formed by an optical metamaterial, a photonic crystal, or an optical antenna array.

The optical modulator may alternatively be formed by a stack of layers, such as a stack of layers of two different types, wherein the two different types of layers have different refractive indices and are alternatingly arranged in the stack. This may form an interference filter providing different responses for light at different incident angles at different wavelengths.

According to an embodiment, the system further comprises a protective layer between the illumination waveguide and the functionalized surface.

The protective layer may protect the illumination waveguide from being damaged. The protective layer may provide protection of the illumination waveguide during manufacturing of an integrated device in which the illumination waveguide is integrated. For instance, the protective layer may be configured to protect the illumination waveguide from being damaged by chemical substances, such as etchants, used during manufacturing of the integrated device or used for providing functionalization of the functionalized surface. The protective layer may also be configured to protect the illumination waveguide from being damaged by any substances used in biosensing, such as tags used for binding to biological entities to be analyzed or buffers used for washing away tags between sequential analyses using different tags.

The protective layer may be formed by a transparent material. The protective layer may at least be transparent to wavelengths of illumination light and the output light in response to illumination. Thus, the protective layer may be configured not to affect illumination of biological entities at the functionalized surface or detection of output light associated with the biological entities.

The protective layer may be arranged on the illumination waveguide only in regions outside the functionalized surface. The protective layer may alternatively be arranged on the illumination waveguide such that the protective layer may be arranged between the illumination waveguide and the functionalized surface. The protective layer may in such case be sufficiently thin, such that an evanescent field of the light in the illumination waveguide may reach the functionalized surface.

According to an embodiment, the system further comprises a microfluidic system configured to transport one or more tags to the functionalized surface for tagging the biological entities bound at binding sites and for providing output of light from the biological entities illuminated by the illumination pattern.

Use of tags facilitates detecting information of interest from the biological entities. A tag may be configured to bind to a distinct molecule for identifying the molecule, or to a distinct functional group for detecting the functional group. The tag may for example be configured to bind to a complementary portion of a molecule, wherein one base or a sequence of bases in the tag binds to complementary base(s) in the molecule.

The tag may further be provided with a component for providing output of light based on illumination. The tag may for instance comprise a fluorophore for providing fluorescence light based on the tag being illuminated by excitation light for exciting the fluorophore.

Use of tags facilitates accurate identification of molecules since it allows for the detection and differentiation of different types of molecules based on the output light from their respective tags. This can be useful in various fields such as biology, chemistry, and medicine where specific molecules need to be identified and analyzed.

Different tags may be configured to bind to different categories of biological entities. Thus, the system may be configured to differentiate light output by different categories of biological entities by the light output by different tags binding to the different categories. The different categories of biological entities further bind to binding sites of different binding site types, further facilitating differentiating output light that is associated with different categories of biological entities.

The system may further be configured to identify a particular biological entity of a category of biological entities. Thus, a binding site of a particular binding site type may have capability to bind different biological entities within the same category. The system may further use sequentially provided tags in order to identify the biological entity that has been bound at the binding site.

The system may incorporate a microfluidic system. This may facilitate transport of the tags to the biological entities being bound at the functionalized surface. The microfluidic system may be integrated with the functionalized surface for providing a compact system.

It should however be realized that tags may be provided in a different manner. For instance, a biological sample to be analyzed may be prepared with tags even before the biological sample is received at the functionalized surface.

The microfluidic system may alternatively be used for transporting biological entities that are prepared with tag(s) bound to the biological entities to the functionalized surface.

According to an embodiment, the microfluidic system is configured to sequentially transport different tags to the functionalized surface and wherein the system is configured to sequentially form different illumination patterns for illuminating each of the binding sites between sequential occasions of transporting tags to the functionalized surface.

As mentioned above, the illumination system may allow changing the illumination pattern for illuminating all of the plurality of binding sites to be illuminated and all parts of the binding sites to be illuminated. The system may be configured to provide illumination of all of the binding sites while the biological entities are tagged using a first specific type of tag. This allows the system to acquire information from all of the binding sites while the first specific type of tag is provided.

The system may, thereafter, using the microfluidic system, remove, such as by washing away, the first specific type of tag and provide a second specific type of tag, different from the first specific type of tag. The system may again change the illumination pattern for acquiring information from all of the binding sites while the second specific type of tag is provided. This may be repeated for a plurality of different types of tags for acquiring extensive information of the biological entities.

Each occasion of transporting tags to the functionalized surface may be referred to as a cycle. During each cycle, at least one type of tag may be transported to the functionalized surface. In a different embodiment, multiple types of tags may be transported to the functionalized surface. For instance, the different types of tags may be configured to emit different wavelengths of light enabling differentiation of responses to different types of tags within the same cycle. The system may further be configured to provide illumination of all of the binding sites using multiple wavelengths, e.g., by providing illumination of all of the binding sites using a first wavelength in a first set of illumination patterns and by providing illumination of all of the binding sites using a second wavelength in a second set of illumination patterns.

Thus, the system may be configured to provide trigger output from different types of tags at different points in time during a cycle. The multiple types of tags may for instance be used for enabling acquisition of information from different biological entities within a same cycle of providing tags to the functionalized surface. For instance, different types of tags within the multiple types of tags may target different biological entities.

According to an embodiment, the system further comprises a processing unit configured to receive data detected by the array of light-detecting areas, wherein the processing unit is configured to generate a reconstructed image of the biological sample based on the received data.

Thus, the responses to illumination detected by the array of light-detecting areas may be used for analyzing the biological entities. The responses may be related to temporal information to interpret detected responses in relation to tags being provided at a point in time at which the responses were detected. The responses may further be related to spatial information to determine a spatial location at the functionalized surface at which the response is provided. This spatial information may for instance be based on a knowledge of a location of an illumination area at the functionalized surface at a point in time at which the responses were detected. It should also be realized that knowledge of the location of the illumination area may not be available. Spatial information of responses may still be determined based on processing responses of a plurality of illumination patterns.

The processing of responses to illumination detected by the array of light-detecting areas may utilize information of the illumination patterns, information of arrangement of binding sites, and/or information of tags being provided to the functionalized surface. Thus, one or more of these types of information may be known for processing of the responses. Reconstruction of information representing the biological entities, such as an image of the biological sample, may not need to utilize each type of information but may rather be possible using only one type of information. However, if all types of information, i.e., information of the illumination patterns, information of arrangement of binding sites, and information of tags being provided to the functionalized surface, are known, reconstruction of information may be efficient.

The processing unit may be configured to use the temporal information and the spatial information for combining a plurality of detected responses to a comprehensive representation of the biological entities. The processing unit may be configured to generate a reconstructed image of the biological sample.

The processing unit may be configured to provide identification of biological entities and locations of the identified biological entities based on processing the detected responses, e.g., by taking the temporal information and the spatial information into account. The processing unit may further determine the reconstructed image for representing a spatial distribution of different biological entities within the biological sample.

Thanks to the plurality of binding sites of different binding site types being distributed across the functionalized surface, the processing unit may be configured to reconstruct an image representing spatial relations between biological entities of a plurality of different categories within the biological sample.

The processing unit may be integrated with the array of light-detecting areas. This implies that image reconstruction of the biological sample may be provided within a compact system. This may also imply that a large amount of data need not be communicated to an external device. However, according to another embodiment, the processing unit may be arranged in a separate device from the array of light-detecting areas. The processing unit may receive data detected by the array of light-detecting areas through wired or wireless communication.

According to an embodiment, the processing unit is configured to generate one or more maps, wherein each map represents a spatial distribution of one or more biological entities in the biological sample.

The image reconstruction may thus provide a representation of the biological sample in form of maps of the biological sample representing one or more characteristics of the biological sample.

For instance, each map may represent a spatial distribution of a category of a biological entity or a particular biological entity within a category. The spatial distribution of a biological entity may be determined based on locations of the binding sites at which the biological entity is bound, tags provided for inducing output of light that is associated with the biological entity, and selective illumination for illuminating the binding sites such that the response from the biological entity may be differentiated from responses from other biological entities (that may be bound at other binding sites).

Each binding site may be configured to bind a single biological entity. Thus, the processing unit may be configured to process the received data to generate maps providing a physical count of biological entities. The presence of a biological entity at a binding site corresponds to a single specimen of the biological entity being bound at the binding site.

According to an embodiment, the functionalized surface comprises at least one antifouling region configured to reduce an adsorption speed or prohibit adsorption of undesired biological entities at the plurality of binding sites, wherein the at least one antifouling region is arranged surrounding one or more binding sites.

The antifouling region may prevent undesired biological entities from reaching the binding sites. For instance, the antifouling region may prevent biological entities from parts of the biological sample that are not being imaged from reaching the binding sites. This implies that the spatial information associated with locations of the binding sites may be maintained and that detection of information of biological entities at the binding sites may be performed with a high signal-to-noise ratio.

The antifouling region may prohibit adsorption or at least reduce an adsorption speed of biological entities at the binding sites. Thus, biological entities approaching the binding sites from a side of the functionalized surface such that the biological entities need to pass the antifouling region may be prevented from reaching binding sites arranged inside the antifouling region or adsorption speed of the undesired biological entities at the binding sites may at least be reduced such that the undesired biological entities may be slowed down to delay a binding of the undesired biological entities to binding sites.

An antifouling region may be arranged surrounding more than one binding site. This may imply that the binding sites surrounded by the antifouling region may be isolated such that biological entities may only reach the binding sites from particular locations within the biological sample (above the binding sites). This may ensure that spatial information associated with the binding sites is linked to a particular location within the biological sample and may enhance the spatial information determined by a plurality of binding sites that are separated by one or more antifouling regions.

According to an embodiment, an antifouling region is arranged surrounding all of the binding sites. Thus, there may be only one antifouling region arranged around a combined area defined by all of the binding sites.

According to an embodiment, a plurality of antifouling regions is provided each surrounding a unique binding site. Thus, each binding site may be associated with an antifouling region for controlling a location in the biological sample from which a biological entity may reach the binding site. The plurality of antifouling regions each surrounding a unique binding site may further be combined with one or more additional antifouling regions, which each surround a plurality of binding sites.

The functionalized surface may be modified or treated in some way to provide the antifouling region. For instance, the functionalized surface may be provided with a coating that provides a desired antifouling function to the functionalized surface. The functionalized surface has been modified or treated in areas corresponding to the antifouling region in order to provide a reduced adsorption speed of undesired biological entities. For instance, the functionalized surface may be treated with self-assembled monolayers (SAM) or other types of coatings made of poly(ethylene oxide), zwitterions, lipids, or other types of molecules to provide antifouling properties.

According to an embodiment, the plurality of binding sites is arranged in a regular array. The plurality of binding sites of each subset may be arranged in a regular sub-array.

The regular array of binding sites may ensure that a distance between neighboring binding sites is identical throughout the array. Further, distances between neighboring binding sites of the same binding site type may also be identical throughout the array.

This facilitates acquisition of information relating to binding sites with an identical resolution throughout the functionalized surface.

According to an embodiment, the system further comprises a plurality of optical units associated with separate parts of the functionalized surface, wherein each optical unit comprises a separate illumination waveguide for individually creating the illumination pattern for illuminating the binding sites of the part of the functionalized surface with which the optical unit is associated.

The optical units may also have separate arrays of light-detecting areas. This may facilitate separate manufacturing of the optical units, wherein each optical unit is an integrated device comprising a functionalized surface, an illumination waveguide, and an array of light-detecting areas.

The optical units may be configured to receive light from a common light source. The optical units may also be configured to be controlled by a common controller for controlling the illumination patterns. However, it should be realized that each optical unit may be associated with a separate light source and/or a separate controller.

It should be realized that the functionalized surface may be a large coherent surface, and different optical units may be associated with different parts of the coherent functionalized surface. Alternatively, the functionalized surface may be formed by separate parts that are not directly connected to each other. In fact, different optical units may be associated with different parts of the functionalized surface that are not arranged in a common plane.

The optical units may be configured to provide different functionalities for performing different measurements on the biological entities bound to the respective part of the functionalized surface. Thus, use of a plurality of optical units may allow performing different measurements in parallel. Alternatively or additionally, the different optical units may be configured to perform similar or identical measurements. Thanks to using a plurality of optical units, a large total area of the functionalized surface may be provided, such that a large capacity of measurements is provided by the system.

The optical units may be mounted on a common substrate, such that the optical units are arranged in parallel on a common plane. The optical units may be associated with a single fluidic interface for providing transport of tags and/or samples for analysis. The use of a plurality of optical units may provide a high throughput of acquiring information relating to biological entities.

Alternatively, the optical units may be mounted in different planes. Optical units may be arranged to face each other, such that separate parts of the functionalized surface are provided by a first and a second optical unit, with binding sites of a first optical unit being configured to face binding sites of a second optical units.

This implies that a microfluidic channel may be arranged between binding sites of two different optical units. This may provide a compact manner of providing a large number of binding sites in relation to a microfluidic channel.

According to an embodiment, the system is integrated in a cartridge which is configured to be introduced into an instrument for analysis of the biological sample.

Thus, the system may be used in a simple manner in an instrument for analysis of biological entities. The instrument may for instance be configured to enable control of transport of biological entities, tags, and any other substances to be used in analysis of the biological entities.

Thanks to the system providing a robust relation between the functionalized surface, the illumination waveguide, and the array of light-detecting areas, the system may be easily used in the instrument for acquiring information of biological entities with high resolution.

The cartridge may for instance comprise the functionalized surface, the illumination system, and the array of light-detecting areas. The cartridge may further comprise a microfluidic system for providing fluidic transport to and from the functionalized surface.

The cartridge need not comprise the controller. Rather, the controller may be provided in the instrument and signals may be communicated from the controller to the cartridge for controlling the illumination pattern.

The cartridge may thus be configured for wired or wireless communication with the instrument. The communication may provide control signals from the instrument to the cartridge for controlling the forming of the illumination pattern. The communication may also or alternatively provide transmitting data acquired by the array of light-detecting areas from the cartridge to the instrument for processing of the data.

The cartridge may also be configured to receive illumination light from a light source arranged in the instrument.

According to an embodiment, the system is part of a higher-level system, such as an analysis instrument, for determining an analysis result relating to the biological sample, such as a higher-level system that is designed to provide an image representation of the biological sample. The system according to the first aspect may thus be configured to measure data that is used for determining the analysis result.

According to a second aspect, there is provided an analysis instrument, which is configured to receive the system according to the first aspect.

Effects and features of this second aspect are largely analogous to those described above in connection with the first aspect. Embodiments mentioned in relation to the first aspect are largely compatible with the second aspect.

High-level functions of the higher-level system can be split into data acquisition, data processing and data management.

Data acquisition function employs one or more modules connected to each other to acquire measurement data, such as the sequencing data, from the biological entity under test. Data acquisition function may comprise modules dedicated to optics, mechanical, fluidic, assay and surface chemistry functions.

Data processing functions may include the processing of data acquired during a measurement. It may comprise of optical readout data and other sensory and experimental inputs originating from detectors, sensors, and user of the system. Data processing module may perform computations to derive quantitative information from the measured data. The quantitative information can include reconstructing an image of the biological sample under measurement. The quantitative information may also include other information related to measurement quality, speed, errors etc.

The data processing may be performed real-time or after an experiment is completed, i.e., after a complete dataset from measurements on the biological sample has been acquired.

Data management function may include recording raw data measured from various sources in the system and also processed data in different forms, such as numerical, graphical, image or text. The data management function may record the data on a local environment such as hard drives attached to the system or may transfer the data to a cloud or server location via a connected network.

According to a third aspect, there is provided a method for spatial analysis of a biological sample, said method comprising: arranging the biological sample on a functionalized surface, wherein the functionalized surface comprises a plurality of binding sites of a plurality of binding site types, wherein the binding sites of a binding site type are distributed across the plurality of binding sites and are configured to bind a biological entity from the biological sample, wherein binding sites of different binding site types are configured to bind different categories of biological entities from the biological sample, wherein the plurality of binding sites comprises a plurality of subsets, wherein each subset comprises at least one binding site or part of a binding site; illuminating the functionalized surface by an illumination pattern for selectively illuminating one or more binding sites at a time, wherein the illuminating comprises controlling the illumination pattern for sequentially illuminating different binding sites; detecting, by an array of light-detecting areas, output light associated with biological entities bound at binding sites and illuminated by the illumination pattern, wherein each light-detecting area is associated with a subset of the binding sites, and wherein the illuminating and detecting provides imaging of the functionalized surface for differentiating responses to the illumination pattern from different binding sites.

Effects and features of this third aspect are largely analogous to those described above in connection with the first and second aspects. Embodiments mentioned in relation to the first and second aspects are largely compatible with the third aspect.

The method enables high resolution of analysis of biological entities that are bound at the binding sites. The resolution may be set using the binding sites and the illumination pattern, which may be used for selectively illuminating a very small illumination area.

The binding sites of different binding site types are configured to bind different categories of biological entities from the biological sample. This implies that multiple aspects of the biological sample may be analyzed using the same functionalized surface. Hence, spatial relationships between different categories of biological entities within the same sample may be determined allowing the system to provide an insight into organization of complex tissues.

The method may allow analyzing the biological entities, such as molecules, of the biological sample in multiple omics fields. Thus, information from multiple omics fields may be acquired from a single specimen of biological sample. For instance, the biological molecules from the biological sample may be analyzed in transcriptome, genome, proteome, metabolome, lipidome, glycome, and/or other omics fields.

According to an embodiment, the method further comprises: transporting tags to the functionalized surface, wherein each tag is configured to bind to a distinct type of the biological entities bound at binding sites, wherein said illuminating of the functionalized surface by the illumination pattern induces output of light from the tags; and determining an identification of the biological entities bound at the binding sites based on detecting of the output light from the tags.

The tags may be used for identifying the biological entities based on whether the tags bind to the biological entity or not. This may be used for providing information of presence of biological entities at a respective binding site.

The method may use a functionalized surface comprising a plurality of binding site types for capturing different biological entities at different binding sites. This may be used for providing spatial information of a biological sample comprising a plurality of cells. The biological sample may be arranged at the functional surface and the analysis of the plurality of biological entities may allow spatial analysis of the biological sample arranged at the functionalized surface. Thus, the biological sample may provide an arrangement of cells in relation to each other. The arrangement of the cells may be analyzed, using the spatial analysis of the biological sample. The spatial analysis may provide analysis of different cells in the biological sample by detecting biological entities (molecules) from respective cells. The analysis of biological entities at particular binding sites may thus provide information of the biological entities present in different parts of the biological sample. The location of the binding sites may be associated with a spatial location in the biological sample.

The method may comprise sequentially transporting different tags to the functionalized surface. This may be used for enabling identifying a plurality of different biological entities based on different tags being configured to bind to different biological entities. Thanks to different tags being sequentially transported to the functionalized surface, differentiation between responses from different tags may be easily performed.

According to an embodiment, the method further comprises reconstructing an image of the biological sample representing a spatial distribution of presence and identifications of the biological entities in the biological sample.

The tags may be used for identifying biological entities that are bound at the binding sites. This may further be used for reconstructing an image representing the distribution of the biological entities within the biological sample.

The binding sites may provide binding of single biological entities per binding site. Thus, the detection of a biological entity at binding sites may be converted to a physical count of the biological entity in a map representing the distribution of the biological entity across the binding sites.

According to an embodiment, arranging of the biological sample on the functionalized surface comprises interfacing the biological sample with reagents for tissue permeabilization and binding biological entities at the binding sites.

Tissue permeabilization may allow biological entities to leave the cells in the biological sample such that the biological entities may diffuse to the functionalized surface and be bound at the functionalized surface. This allows determination of spatial information of the biological sample by extracting biological entities from the biological sample.

The method may comprise providing tags to the biological entities in the biological sample before the biological entities leave the cells and are bound at the functionalized surface. Alternatively, tags are provided when the biological entities have been bound at the functionalized surface.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view of a system according to an embodiment.
Fig. 2 is a cross-sectional view of a part of the system according to an embodiment.
Fig. 3 is a schematic view of binding sites of a subset arranged at a functionalized surface of the system.
Fig. 4 is a schematic view of packaging of the system according to an embodiment.
Fig. 5 is a schematic view of the system according to an embodiment, illustrating input of light to the system.
Fig. 6 is a schematic view of the system according to an embodiment, illustrating an alternative manner of inputting of light to the system.
Fig. 7 is a schematic view of the system according to an embodiment, illustrating use of a plurality of optical units.
Fig. 8 is a flow chart of a method according to an embodiment.

### Detailed description

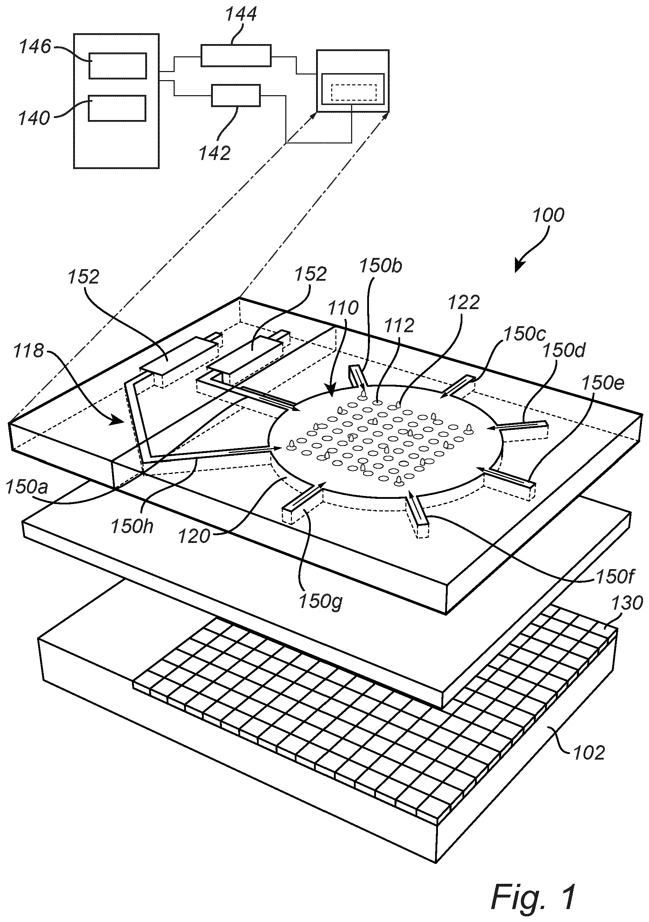

Referring now to Fig. 1, a system 100 for spatial analysis of a biological sample will be described. The system 100 comprises a functionalized surface 110 configured to bind biological entities to be analyzed and an illumination system 118 for providing illumination of the biological entities being bound at the functionalized surface 110.

The functionalized surface 110 is a surface that has been treated in selective locations for facilitating that particular biological entities are bound at particular locations on the functionalized surface 110. The functionalized surface 110 may comprise a plurality of binding sites 112. Each binding site 112 may be defined at the functionalized surface 110 as a portion of the surface being treated for facilitating binding of a biological entity. For instance, the binding site 112 may be chemically treated providing a capturing probe that may be configured to bind a particular biological entity.

The binding sites 112 may be relatively small. The functionalized surface 110 may thus be provided with a large number of binding sites 112 and may also be provided with a high spatial resolution of separate binding sites 112 on the functionalized surface 110. This may be used for providing spatial resolution of information of a biological sample in that biological entities binding to a particular binding site 112 may provide information relating to a corresponding spatial location in the biological sample.

Thanks to the binding sites 112 being small, each binding site 112 may further be configured to bind a single biological entity. This may facilitate determining detailed information of the biological entity, such as for sequencing of the biological entity. However, it should be realized that the binding sites 112 may alternatively be configured to bind more than one biological entity.

A size of the binding sites 112 may for instance be smaller than 1000 nm, such as in a range of 1 - 750 nm, such as in a range of 5 - 500 nm. This may be useful for providing high resolution of information relating to the biological entities. The size of the binding site 112 may correspond to a diameter of the binding site 112 or a length of a side of the binding site 112 (e.g., a length of a side of a square-sized binding site).

The system 100 further comprises an illumination system 118. The illumination system 118 is arranged in relation to the functionalized surface 110 for illuminating the functionalized surface 110. The illumination system 100 is configured to form an illumination pattern at the functionalized surface 110 for selectively illuminating one or more binding sites 112 at a time.

Thus, the illumination system 118 is configured to provide an illumination pattern that has a variation over the functionalized surface 110. The illumination pattern may provide a spatial frequency of features of the illumination pattern in selective illumination of one or more binding sites 112. The spatial frequency of the illumination pattern may contribute to a spatial resolution of acquiring information of the biological entities bound at the binding sites 112.

The illumination system 118 may be configured in various manners for forming the illumination pattern on the functionalized surface 110. The illumination system 118 may for instance be configured to illuminate the functionalized surface 110 so as to be incident on the functionalized surface from a side at which the biological entities are bound. The illumination system 118 may thus be configured to provide illumination through the biological sample for reaching the functionalized surface 110. The illumination system 118 may be configured to form the illumination pattern on the functionalized surface 110 based on interference of one or more light beams.

The illumination system 118 may comprise one or more optical components for controlling the illumination pattern.

The illumination system 118 is further configured to control the illumination pattern for changing the illumination pattern in a sequence of illumination patterns. This allows the illumination pattern to be sequentially changed such that the entire functionalized surface 110 is illuminated or at least desired parts of the functionalized surface 110 are illuminated through the sequence of illumination patterns.

According to an embodiment, the illumination system 118 comprises an illumination waveguide 120. The illumination waveguide 120 is configured to propagate illumination light to the functionalized surface 110. The illumination waveguide 120 is integrated with the functionalized surface 110. This implies that a robust relation between the illumination waveguide 120 and the functionalized surface 110 is provided. This ensures that control of propagation of light in the illumination waveguide 120 may provide an accurate control of parts of the functionalized surface 110 being illuminated.

The functionalized surface 110 may be configured to face a sample such that the sample is provided at the functionalized surface 110. The functionalized surface 110 may thus be configured to receive the biological sample.

The illumination waveguide 120 may be arranged in an opposite direction in relation to the functionalized surface 110 compared to a direction that the functionalized surface 110 faces. The illumination waveguide 120 may thus be referred to as being below the functionalized surface 110.

The illumination waveguide 120 may be configured to propagate illumination light through total internal reflection in the illumination waveguide 120. The illumination waveguide 120 may extend in parallel with the functionalized surface 120 for propagating illumination light in parallel with the functionalized surface 120.

The total internal reflection of light implies that there is an evanescent field of light outside the illumination waveguide 120 at a location in which total internal reflection occurs. The evanescent field is based on a small portion of light escaping a surface in which total internal reflection occurs and intensity of the light will very quickly decrease with distance from the surface. This evanescent field may interact with an object arranged close to a surface of the illumination waveguide 120 and may thus be used for illuminating an object, such that the illumination waveguide 120 provides selective illumination only of a region very close to the surface (in the order of 50 - 100 nm), such as biological entities bound at the binding sites 112 of the functionalized surface 110.

The illumination waveguide 120 may be configured to propagate one or more light waves for forming an illumination pattern based on interference of the one or more light waves. Thus, propagation of illumination light in the illumination waveguide 120 may be controlled so as to determine an illumination pattern. The illumination pattern may comprise at least one illumination area 122 that is configured to selectively illuminate one or more binding sites 112 of the functionalized surface. The illumination pattern may be defined by different degrees of constructive interference and destructive interference of the one or more light waves, such that distinct illumination area(s) 122 may be defined for providing illumination of one or more binding sites 112 while areas outside the illumination area(s) are not provided with illumination light.

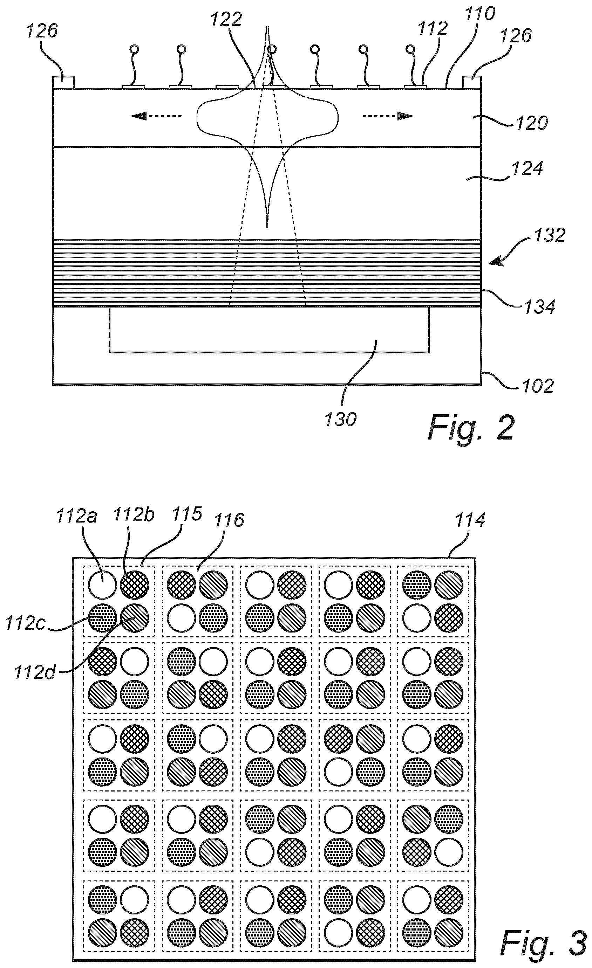

Referring now to Fig. 2, showing a cross-section of the system 100, the illumination waveguide 120 may be arranged between a bottom cladding layer 124 and a top cladding layer 126. The bottom cladding layer 124 may be formed from a material having a relatively low refractive index compared to a refractive index of the illumination waveguide 120 so as to ensure that light propagating through the illumination waveguide 120 is subject to total internal reflection in an interface between the illumination waveguide 120 and the bottom cladding layer 124.

Similarly, the top cladding layer 126 may also be formed from a material having a relatively low refractive index so as to ensure that light propagating through the illumination waveguide 120 is subject to total internal reflection in an interface between the illumination waveguide 120 and the top cladding layer 126. The bottom cladding layer 124 and the top cladding layer 126 may be formed from the same material.

The top cladding layer 126 may be provided with one or more openings above the illumination waveguide 120. This implies that the functionalized surface 110 may be provided by a surface of the illumination waveguide 120 at the opening in the top cladding layer 126. However, it should be realized that a surface of the top cladding layer 126 may form the functionalized surface 110, with the top cladding layer 126 being thin to allow the evanescent field of illumination light propagating in the illumination waveguide 120 to reach the functionalized surface 110.

The opening in the top cladding layer 126 may be larger than an area of the illumination waveguide 120 which is used for illumination of the functionalized surface 110. This implies that any diffraction or other influence on light propagating through the illumination waveguide 110 at an edge of the top cladding layer 126 facing the opening may be diminished before light reaches a portion of the functionalized surface 110 to be illuminated.

The illumination of the functionalized surface 110 by the illumination area 122 of the illumination pattern may induce output of light associated with a biological entity that is bound at a binding site 112 of the functionalized surface. The output of light may be provided by the biological entity itself or by a tag that may be bound to the biological entity. For instance, the output of light may be induced through photoluminescence, such as through fluorescence of light.

The illumination area 122 may induce output of light in a specific point at the functionalized surface 110. This may cause output of light to propagate according to a point spread function (PSF). The output light may propagate from the functionalized surface 110 through the illumination waveguide 120 towards an array of light-detecting areas 130 for detection of the output light.

The output light may propagate in a direction close to a normal of the functionalized surface 110. This implies that the output light may be incident on surfaces of the illumination waveguide 120 at an incident angle allowing the output light to pass the illumination waveguide 120.

Each light-detecting area 130 may be configured to generate a response, such as an electric charge, in proportion to light incident on the light-detecting area 130. Thus, the light-detecting area 130 may generate a measurement of intensity of the output light associated with a biological entity. Thanks to using the illumination pattern for illuminating the object, the light detected by the light-detecting area 130 may be associated with a portion of the functionalized surface 110 defined by the illumination area 122. Thus, a size of the illumination area 122 may define a resolution of acquiring of information by the array of light-detecting areas 130.

The illumination pattern may be configured to provide structured illumination defining a resolution of acquiring information from the functionalized surface 110. The structured illumination may thus provide a sub-pixel resolution, i.e., the illumination area 122 gives rise to a PSF, which in a plane of the array of light-detecting areas 130 is smaller than a size of a single light-detecting area 130. Thus, the illumination area 122 may be moved for illuminating different parts of the functionalized surface 110 while the response associated with biological entities at different locations of the illumination area 122 may be acquired by the same light-detecting area 130.

The array of light-detecting areas 130 may for example be implemented as a charge-coupled device (CCD) or a complementary metal-oxide-semiconductor (CMOS) image sensor, being arranged for frontside or backside illumination. The array of light-detecting areas 130 may be configured to provide event-based imaging. Thus, only when a light-detecting area 130 captures a signal variation exceeding a certain threshold, read-out of a value from the light-detecting area 130 will be triggered. Analog output from the light-detecting areas 130 may pass an analog-to-digital converter, such that the array of light-detecting areas 130 may output a digital representation of detected light. The digital representation is then suited to be transferred to other entities for processing the detected light and/or being processed within the system 100.

The array of light-detecting areas 130 may be integrated with the illumination waveguide 120, for instance being formed on a common substrate 102. This implies that the array of light-detecting areas 130 may be arranged close to the illumination waveguide 120 and, hence, also close to the functionalized surface 110 from which output light is to be detected.

The illumination waveguide 120 may be monolithically integrated on the substrate 102 on which the array of light-detecting areas 130 is formed. Alternatively, the array of light-detecting areas 130 and the illumination waveguide 120 may be separately manufactured in two separate physical units, which may then be bonded to each other.

According to yet another alternative, the array of light-detecting areas 130 is not integrated with the illumination waveguide 120. The array of light-detecting areas 130 may be arranged in relation to the illumination waveguide 120 and the functionalized surface 110 to provide a robust relation such that output light associated with biological entities bound at binding sites 112 of the functionalized surface 110 may be detected by an associated light-detecting area 130.

Referring again to Fig. 1, the system 100 may comprise a plurality of input waveguides 150a-h. Each of the plurality of input waveguides 150a-h may propagate light and be configured to couple light into the illumination waveguide 120. Thus, the illumination waveguide 120 may receive a plurality of light waves from the plurality of input waveguides 150a-h. It should be realized that the number of input waveguides 150a-h may be selected dependent on need for controlling the illumination pattern in the illumination waveguide 120.

Light coupled into the illumination waveguide 120 will interact in the illumination waveguide 120 to form an illumination pattern based on interference of light. The illumination pattern formed will be dependent on the light waves input into the illumination waveguide 120 and the relation between these light waves. If a single input waveguide 150 is used, light may enter the illumination waveguide 120 at a first end and may be reflected by a mirror at an opposite end of the illumination waveguide 120 such that an interference between light from the input waveguide 150 and reflected light may form the illumination pattern. It should also be realized that a system 100 comprising a plurality of input waveguides 150a-h may further comprise one or more mirrors for reflecting light back into the illumination waveguide 120.

The interference of light in the illumination waveguide 120 may form a distinct illumination pattern based on the relation between light waves propagating in the illumination waveguide 120. Thus, by maintaining constant relations between light waves, distinct illumination areas 122 of the illumination pattern may be defined. The relations between light waves may be changed for changing locations of the illumination areas 122.

The interference of light in the illumination waveguide 120 may thus be controlled by affecting a light wave in at least one of the input waveguides 150 and/or affecting reflection of light by at least one mirror. This implies that a relation between light waves propagating in the illumination waveguide 120 is changed such that the illumination pattern in the illumination waveguide 120 will also change.

The light propagating in the input waveguide 150 may be controlled by controlling light being provided into the input waveguide 150. For instance, a light source providing light to the input waveguide 150 may be controlled for controlling light being provided into the input waveguide 150. Alternatively, the input waveguide 150 may be associated with an influencing device 152, which may be configured to affect propagation of light in the input waveguide 150.

To obtain a desired illumination pattern, one or more of the phase, wavelength, and intensity of light in the input waveguides 150a-h can be controlled. By adjusting phase, wavelength, and/or intensity of light in the input waveguides 150a-h, the illumination pattern may be changed. The properties to be used for the light being input into the illumination waveguide 120 may be determined using for instance holographic tweezer techniques which are adopted for two-dimensional waveguides.

Holographic tweezer techniques often use the Fourier transform (FT-) based Gerchberg-Saxton (GS) algorithm, an iterative search procedure for obtaining a hologram phase that can yield the desired intensity distribution. Another approach can be to consider the input waveguides 150a-h and the illumination waveguide 120 as a waveguide phased array analog.

Tuning of a phase, intensity, and/or wavelength of light in the input waveguide 150 may be achieved in a number of different manners. For instance, the influencing device 152 may be used for controlling phase, intensity, and/or wavelength. Thus, the influencing device 152 may control a characteristic which affects propagation of light in the input waveguide 150.

For instance, the influencing device 152 may provide a thermal modulation of the light wave in the input waveguide 150. Thus, by heating of a local environment in which the input waveguide 150 is arranged, a phase, intensity, or wavelength of light in the input waveguide 150 may be tuned. The heating of the local environment may for instance be used for changing an optical path length of the input waveguide 150, such that a thermal phase shifter is provided.

Alternatively, the influencing device 152 may affect propagation of light in the input waveguide 150 through an opto-electro-mechanical effect. For instance, the input waveguide 150 may be at least partly arranged on a membrane, which may be configured to flex in dependence of an electrical signal, e.g., affecting a support of the membrane. A length of the input waveguide 150 may thus be changed due to a change of shape of the membrane. According to another embodiment, a membrane may be arranged in close relation to the input waveguide 150 and may be configured to be selectively pulled towards the input waveguide 150, e.g. by electrostatically pulling the membrane, such that an effective refractive index of the input waveguide 150 may be changed in dependence of a relation between the membrane and the input waveguide 150.

According to yet another alternative, the influencing device 152 may affect propagation of light in the input waveguide 150 by applying an electric or magnetic field to the input waveguide 150. Hence, the influencing device 152 may be configured to provide an electric or magnetic field in a local environment in which the input waveguide 150 is arranged. Then, a refractive index of the input waveguide 150 may be changed in dependence of the applied electric or magnetic field.

It should be realized that the influencing device 152 may be configured to affect propagation of light in the input waveguide 150 in other manners, as known to the person skilled in the art. Also, the influencing device 152 may comprise a plurality of parts which may provide a combined effect on the propagation of light in the input waveguide 150.

The input waveguide 150 and the influencing device 152 may be arranged on the common substrate 102 so that the illumination pattern may be controlled by components arranged on the common substrate 102 facilitating a compact system 100.

The functionalized surface 110 may comprise binding sites 112 of a plurality of binding site types. The binding sites 112 of different binding site types are configured to bind different categories of biological entities from the biological sample. This implies that multiple aspects of the biological sample may be analyzed using the same functionalized surface.

The biological entity may be a molecule, such as a deoxyribonucleic acid (DNA) molecule, a ribonucleic acid (RNA) molecule, a protein, a peptide, a metabolite, a lipid, a sugar, a vitamin, a hormone, an aptamer, a small-molecule drug, or other molecular features. The biological entity may alternatively be a complex entity comprising two or more molecules bound to each other. The biological entity may be a complex entity of an antibody and an antigen, an enzyme and a substrate, a protein and another protein, a protein and a small molecule, a nucleic acid with epigenetic modifications (e.g., DNA methylation, histone modifications), proteins with glycosylation patterns, an aptamer and a target molecule, a DNA origami structure, a protein origami structure, or other complexes.

The different categories of biological entities may relate to any biological entities present in a biological sample, such as any molecules present in the biological sample. For instance, the different binding site types of the plurality of binding site types may be configured to bind molecules in two or more categories selected from a group comprising DNA, RNA, proteins, metabolites, lipids, sugars, vitamins, hormones, and aptamers. In an embodiment, DNA, RNA, proteins, and metabolites are four different categories of biological entities. However, it should be realized that other combinations of different categories of biological entities could be used and that different numbers of binding site types binding molecules of different categories may be provided.

The binding sites 112 may each be configured to capture a particular category of molecules. Thus, the binding sites 112 may be provided with polyadenylation (poly-A) to be configured to bind to RNA, or functional groups to bind to proteins or DNA. The binding sites 112 may thus bind a particular category of molecules that may thereafter be identified, sequenced, and/or characterized.

Hence, spatial relationships between different categories of biological entities within the same sample may be determined allowing the system 100 to provide an insight into organization of complex tissues.

This implies that the system 100 may allow analyzing the biological entities, such as molecules, of the biological sample in multiple omics fields. Thus, information from multiple omics fields may be acquired from a single specimen of biological sample. For instance, the biological molecules from the biological sample may be analyzed in transcriptome, genome, proteome, metabolome, lipidome, glycome, and/or other omics fields.

Each binding site 112 may be provided with a binding element configured to capture a molecule, such as configured to capture a particular category of molecules. Different binding elements are configured to capture different molecules. Thus, the binding sites 112 of different binding site types may be provided with different binding elements for ensuring that different categories of molecules are bound at different binding sites.

The binding sites 112 may also or alternatively be provided with binding elements that may provide a specificity in binding of molecules. For instance, binding elements that may be provided at the binding sites 112 may include single-stranded DNA/RNA, which may be hybridized with complementary single-stranded DNA/RNA captured at the binding site 112; antibody (for proteins) or antigen (for small biomolecules) which may bind with complementary antigen or antibody, respectively; DNA aptamer; streptavidin for binding biotin or vice versa; designed DNA origami structures; protein origami structures; molecular imprinted polymers (MIP), etc.

However, the binding sites 112 need not necessarily be provided with binding elements that may directly capture the biological entities for binding the biological entities. For instance, a binding site 112 may be prepared for specifically receiving a binding element. Thus, the binding site 112 may be provided with a receiver element, which may be configured to bind a particular binding element to the binding site 112. This implies that the binding element may be provided during or shortly prior to analysis of the biological sample for providing respective binding elements to respective binding sites 112. The receiver element at a binding site 112 may thus bind a specific binding element to the binding site 112 and the specific binding element may in turn be configured to capture and bind specific biological entities to the binding site 112.

The binding sites 112 may provide spatial resolution of information of a biological sample. The binding sites 112 may receive biological entities from a part of the biological sample such that the biological entities bound at a binding site 112 may represent the biological entities present at a corresponding location in the biological sample.

The spatial resolution of information provided by the binding sites 112 may further be extracted using the illumination pattern and the detection of light by the array of light-detecting areas 130. Each light-detecting area 130 may be configured to receive light output by biological entities being bound at a subset of the binding sites 112. The system 100 may be configured to differentiate information from different binding sites 112 by temporally controlling from which binding sites 112 responses to illumination light are received and spatially controlling which binding sites 112 are illuminated.

Each subset may comprise a plurality of binding sites 112. In other words, each light-detecting area 130 may be configured to receive light from a plurality of binding sites 112. The illumination system 118 may be configured to control the illumination pattern such that light associated with biological entities bound to a single binding site 112 of the subset is output at a particular point in time. This may ensure that light detected by the light-detecting area 130 may be determined to originate from a single binding site 112. The illumination system 118 may alternatively be configured to provide a sequence of illumination patterns that allow light output from respective binding sites to be determined.

As mentioned above, the functionalized surface 110 comprises a plurality of binding sites 112 of different binding site types. Thus, different categories of biological entities are bound by binding sites 112 of different binding site types. The different categories of biological entities may be controlled to output light at different points in time.

For instance, the system 100 may be configured to provide tags that bind to the biological entities. Different tags may be configured to provide different interaction with illumination light. For instance, the tags may comprise fluorophores and different tags may comprise different fluorophores, such that a wavelength of the illumination light may activate different tags. In addition, different tags may bind to different categories of biological entities. Thus, the system 100 may be configured to provide an illumination pattern that provides simultaneous illumination of a plurality of binding sites 112 of different binding site types and a response may only be provided from one of the binding sites 112.

Different tags may also be sequentially provided, such that a single type of tag may be bound to the biological entities during illumination.

Each subset may comprise a plurality of binding sites 112, wherein the binding sites 112 are of different binding site types. This may facilitate differentiating responses to illumination light from different binding sites 112. The system 100 may ensure that light may only be output from binding sites 112 of a single binding site type at a time, e.g., by using sequentially provided tags targeting biological entities bound at different binding site types or by using tags that provide different responses to illumination light, such that only one type of tag will provide a response to a particular illumination.

Referring now to Fig. 3, a subset 114 of binding sites 112 is illustrated. A single light-detecting area 130 is associated with the subset 114. Thus, the light-detecting area 130 will receive output light associated with biological entities bound to any binding site 112 with the subset 114.

As shown in Fig. 3, the subset 114 comprises a plurality of binding sites 112 of each binding site type. Thus, the system 100 may provide spatial resolution of information relating to each binding site type based on the arrangement of the binding sites 112 on the functionalized surface 110. In addition, the illumination system 118 may enable differentiation of information from different binding sites 112 in the subset 114. Thus, information of high spatial resolution may be acquired without a need for having a high resolution of the arrangement of the light-detecting areas 130.

As shown in Fig. 3, four different binding site types may be provided. It should be realized that the system 100 may have fewer or larger number of binding site types. The subset 114 in Fig. 3 comprises first binding sites 112a of a first binding site type, second binding sites 112b of a second binding site type, third binding sites 112c of a third binding site type, and fourth binding sites 112d of a fourth binding site type. The different binding site types may enable analysis in four omics fields.

For instance, the first binding sites 112a may be configured to bind DNA molecules for providing genome analysis, the second binding sites 112b may be configured to bind RNA molecules for providing transcriptome analysis, the third binding sites 112c may be configured to bind protein molecules for providing proteome analysis, and the fourth binding sites 112d may be configured to bind metabolite molecules for providing metabolome analysis.

The binding sites 112 may be arranged in regular manner. Thus, within the subset 114, a plurality of unit cells 115 may be provided. Each unit cell 115 may comprise one binding site 112 of each binding site type. In Fig. 3, each unit cell 115 comprises a first binding site 112a, a second binding site 112b, a third binding site 112c, and a fourth binding site 112d.

The binding sites 112a-d may be arranged in an identical manner in each of the unit cells 115. Alternatively, the binding sites 112a-d may be arranged in a random order, such that different arrangements of the binding sites 112a-d may be provided in different unit cells 115. As yet another alternative, the binding sites 112a-d of different binding site types may not necessarily be arranged in unit cells. Rather, a subset 114 may comprise a number of binding sites 112a-d per binding site type, and the binding sites 112 may be arranged in a random order within the subset 114.

According to an embodiment, the subset 114 comprises at least four unit cells 115 arranged in rows and columns, such as unit cells 115 arranged in an arrangement between 2x2 to 10x10 within the subset 114. It should also be realized that the binding sites 112 may be arranged to define unit cells 115 with a rectangular arrangement but other arrangements are also possible, such as hexagonal arrangements of unit cells 115, or non-periodic or quasi-period arrangement of unit cells 115.

The functionalized surface 110 may further comprise at least one antifouling region 116. An antifouling region may be arranged surrounding an entire area formed by the plurality of binding sites 112. This may be used for preventing fouling of binding sites 112 from an exterior to the area of the plurality of binding sites 112.

Antifouling regions 116 may further be provided between binding sites 112. The antifouling regions 116 may surround one or more binding sites 112. For instance, an antifouling region 116 may surround a subset 114 or a unit cell 115, or a single binding site 112.

The antifouling region 116 may provide control of a spatial location from which biological entities being bound to the binding sites 112 are received. Thus, the antifouling region 116 may ensure that the biological entities being bound at a binding site 112 may be related to information of a corresponding location in the biological sample.

The antifouling region 116 may be configured to prohibit adsorption of undesired biological entities at the binding sites 112 or at least to reduce an adsorption speed.

The functionalized surface 110 may be modified or treated in some way to provide the antifouling region 116. For instance, the functionalized surface 110 may be provided with a coating that provides a desired antifouling function to the functionalized surface 110. The functionalized surface 110 has been modified or treated in areas corresponding to the antifouling region 116 in order to provide at least a reduced adsorption speed of undesired biological entities. For instance, the functionalized surface 110 may be treated with self-assembled monolayers (SAM) or other types of coatings made of poly(ethylene oxide), zwitterions, lipids or other types of molecules to provide antifouling properties.

The use of antifouling regions 116 may facilitate acquiring spatial information of the biological sample. The use of antifouling regions 116 may imply that information may be acquired with a higher signal-to-noise ratio compared to no antifouling regions 116 being provided.

The system 100 may be configured to detect biological entities being bound at the binding sites 112. The system 100 may be configured to determine information with high sensitivity and may be configured to detect information based on a single biological entity being bound at respective binding sites 112. Thus, there may be no need of an artificial amplification for increasing a number of biological entities. This also implies that the system 100 is not affected by amplification bias due to different molecules having different amplification efficiency.

The system 100 may be configured to differentiate between different biological entities within a same category by using tags. For instance, by sequentially providing different tags which may bind to different specific biological entities within the same category, the system 100 may enable detection of the different specific biological entities being bound to different binding sites 112 of the same binding site type. Alternatively, the binding sites 112 of the same binding site type may be configured to specifically bind a particular biological entity such that binding sites 112 of the same binding site type may be selective to bind different biological entities of the same category. This may be used for differentiating information relating to different biological entities of the same category.

The illumination system 118 is configured to form an illumination pattern at the functionalized surface 110 for selectively illuminating one or more binding sites 112 at a time. This will now be further discussed in relation to the illumination system 118 comprising an illumination waveguide 120. However, it should be realized that the illumination system 118 may provide illumination of the functionalized surface 110 in a different manner for selectively illuminating the binding sites 112.

As best shown in Fig. 2, the illumination pattern may comprise at least one illumination area 122, which may be defined by constructive interference of light waves propagating in the illumination waveguide 120. The illumination area 122 may provide selective illumination of at least one portion of the functionalized surface 110. The illumination area 122 may be defined with a high resolution. The illumination area 122 may be configured to provide structured illumination such that the resolution of acquiring information from the functionalized surface 110 may be set by the illumination pattern.

The illumination area 122 may for instance be configured to illuminate one or more binding sites 112 or a part of a binding site 112. The illumination area 122 may thus selectively illuminate a biological entity bound at a binding site 112 or part of the biological entity.

The changing of the illumination pattern may move the at least one illumination area 122 in relation to the functionalized surface 110 such that the illumination area 122 is scanned over the binding sites 112 for acquiring information from all of the binding sites 112.

The illumination area 122 may provide a selective illumination of the functionalized surface 110 in two dimensions. The at least one illumination area 122 may thus have an extension in two dimensions and the smallest extension in these two dimensions may have a size in the range of 100 nm - 10 µm, such as in a range of 100 nm - 10 µm, such as in a range of 100 - 1000 nm. This implies that the at least one illumination area 122 may selectively illuminate a portion of the functionalized surface 110 of a corresponding size providing a spatial resolution of acquiring of illumination of the functionalized surface 110. Information acquired from a plurality of illumination patterns may further be used for extracting information with the spatial resolution of individual illumination patterns of with even higher spatial resolution.

The at least one illumination area 122 may have a shape of a circular or elliptical spot. This implies that the illumination area 122 may select a portion of the functionalized surface 110 in two dimensions such that a resolution in two dimensions may be provided by the at least one illumination area 122. However, it should be realized that the at least one illumination area 122 may alternatively have a shape of a line, such that a selective illumination of the functionalized surface 110 is provided only in one dimension.

The changing of the illumination pattern may be configured to scan the one or more spots of the illumination pattern over the functionalized surface 110. A throughput of the system 100 may then be defined by a scanning speed and a number of illumination areas 122 in the illumination pattern. However, the throughput may further be limited by an integration time for acquiring frames by the array of light-detecting areas 130, which integration time may be longer than an available speed of scanning the illumination pattern.

The illumination pattern may be formed as a regular pattern of a plurality of illumination areas 122, e.g., in form of spots. The illumination pattern may comprise a periodic pattern of illumination areas 122, which periodic pattern is moved over the functionalized surface 100 for illuminating each of the binding sites 112. The illumination areas 122 of the different illumination patterns as the illumination pattern is changed may in combination cover a combined area of all of the binding sites 112. In an embodiment, the periodic pattern may be a periodic lattice of equilateral triangles, which may allow adjacent illumination areas 122 within an illumination pattern to be close to each other while avoiding crosstalk between output light induced by different illumination areas 122. Thus, a number of translations required to cover the functionalized surface 110 may be minimized.

Rather than scanning the illumination area 122 in relation to the functionalized surface 110 for different illumination patterns, the illumination pattern may be changed between a number of set-ups which together cover the combined area of the binding sites 112. For instance, if an illumination pattern is formed with a plurality of lines, an angle and/or translational position of the lines may be changed between subsequent illumination patterns. Using a number of different angles and positions of the lines, the combined area of the binding sites 112 may be illuminated. In addition, processing of information detected by the array of light-detecting areas 130 may take into account relations between different illumination patterns for determining high-resolution information relating to biological entities that are bound at the binding sites 112.

Referring again to Fig. 1, the system 100 may further comprise a controller 140 for controlling the illumination pattern formed by the illumination system 118, such as for controlling the illumination pattern formed in the illumination waveguide 120. The controller 140 may be configured to change the illumination pattern.

The controller 140 may be configured to provide control signals in order to affect a light wave being input to the illumination waveguide 120 for controlling the illumination pattern in the illumination waveguide 120.

The controller 140 may send the control signal to an external light source, so as to control the light emitted by the external light source. Light from the external light source may then be coupled to an input waveguide 150 and further coupled from the input waveguide 150 into the illumination waveguide 120.

The controller 140 may alternatively or additionally send the control signal to the influencing device(s) 152, which may be configured to act on the input waveguide(s) 150 so as to affect propagation of light in the input waveguide(s) 150.

The controller 140 may be configured to tune at least one of a phase, intensity, or wavelength of light in the input waveguide 150 for affecting light input into the illumination waveguide 120 and the interference pattern formed therein.

The controller 140 may be configured to control the one or more light waves propagating in the illumination waveguide 120 for sequentially forming different illumination patterns comprising illumination areas 122 in different positions to illuminate different binding sites 112 or different parts of binding sites 112. Thus, the controller 140 may ensure that a sequence of illumination patterns is formed for illuminating the binding sites 112.

The controller 140 may be configured to synchronize changing of the illumination pattern with a frame rate of the array of light-detecting areas 130. The controller 140 may thus ensure that each frame is acquired by the array of light-detecting areas 130 in relation to a desired illumination pattern.

The controller 140 may be configured to continuously change the illumination pattern such that illumination areas 122 of the illumination pattern may slowly move while the frames are acquired. This may still allow associating the detected output light with a location at the functionalized surface 110. Alternatively, the controller 140 may be configured to change the illumination pattern in steps, such that the illumination areas 122 of the illumination pattern are moved in discrete steps between subsequent frames.

The controller 140 may be pre-programmed to provide a sequence of control signals so as to form a sequence of illumination patterns in the illumination waveguide 120. Alternatively, the controller 140 may be configured to calculate a relation between light waves input to the illumination waveguide 120 for forming a desired illumination pattern and then apply corresponding control signals for controlling forming of the desired illumination pattern.

The controller 140 may be configured to control influencing devices 152 for controlling the illumination pattern in the illumination waveguide 120 and may also be configured to control acquiring of frames by the array of light-detecting areas 130. Alternatively, the acquiring of frames by the array of light-detecting areas 130 may be separately controlled.

The controller 140 may be implemented in hardware or as any combination of software and hardware. For instance, the controller 140 may comprise a central processing unit (CPU), or a graphical processing unit (GPU), comprising software for providing functionality of the controller 140 in a general-purpose processor. Alternatively, the controller 140 may be implemented as firmware arranged in an embedded system. As a further alternative, the controller 140 may be implemented as a special-purpose circuitry for providing specific logical operations. Thus, the controller 140 may be provided in the form of an application-specific integrated circuit (ASIC), an application-specific instruction-set processor (ASIP) or a field-programmable gate array (FPGA).

The controller 140 may be configured to send the control signals to the influencing devices 152 via an integrated circuit 142 providing driver electronics for providing electrical signals to control illumination.

The controller 140 and the integrated circuit 142 may be formed on the common substrate 102. Alternatively, the controller 140 and the integrated circuit 142 may be mounted together with the common substrate 102 on a common carrier, which may for instance be formed by a printed circuit board.

Each light-detecting area 130 is associated with a subset of binding sites 112. The light-detecting area 130 may be configured to receive output light from biological entities that are bound at the binding sites 112 of the subset. Thus, the plurality of binding sites 112 may comprise a plurality of subsets, wherein each subset may be associated with a respective light-detecting area 130.

The light-detecting area 130 may be associated with a single binding site 112 or a plurality of binding sites 112. Thus, each subset of binding sites 112 comprises at least one binding site 112.

Each subset may comprise a plurality of binding sites 112 of each binding site type. This implies that a single light-detecting area 120 may be configured to detect output light from a plurality of binding sites 112. The system 100 may provide resolution of acquiring of information by using small binding sites 112 that may be arranged close to each other and by providing small illumination areas 122 of the illumination pattern. Thus, a high resolution may be achieved without requiring small light-detecting areas 130.

The system 100 is configured to form the illumination pattern such that a single illumination area 122 is provided in a subset of the binding sites 112. This implies that each illumination area 122 may be associated with a unique light-detecting area 130. The light-detecting area 130 may detect output light that is induced by a single illumination area 122. This may facilitate associating the detected light with a particular binding site.

The illumination pattern may further be controlled such that an illumination area 122 illuminates a single binding site of a same binding site type. Thus, even though the illumination area 122 may extend across a binding site or even a plurality of binding sites, the illumination area 122 may be configured to only extend over a single binding site 122 of the same binding site type. In some embodiments, the illumination pattern may be controlled such that an illumination area 122 illuminates more than one binding site of the same binding site type.

Binding sites 112 of different binding site types may be configured to bind different biological entities. The system 100 may be configured to provide tags that bind to different biological entities. Different tags may be provided at different points in time, e.g., in a sequential manner. Thus, light may be output from a particular biological entity at a point in time when a tag associated with the biological entity is provided. This implies that light may not be output from binding sites 112 of different binding site types at the same time. Hence, the illumination area 122 may extend over binding sites 112 of different binding site types while the detected light may still be determined as originating from a particular binding site 112.

As mentioned above, the illumination pattern may comprise a plurality of illumination areas 122. Each illumination area 122 may be arranged in a unique subset of the plurality of binding sites 112. This implies that the output light induced by the illumination provided in the illumination area 122 may be provided to a unique light-detecting area 130. Thus, the light detected by each light-detecting area 130 may be induced by a unique illumination area 122 such that the detected light may be determined as originating from a particular binding site 112 illuminated by the unique illumination area 122.

The array of light-detecting areas 130 may be connected to circuitry 144, e.g., read-out circuitry for reading out information of detected intensities of light from the array of light-detecting areas 130. The circuitry 144 may for instance be arranged on the common carrier. The read-out circuitry 144 may be configured to convert analog information detected by the array of light-detecting areas 130 into digital domain.

The system 100 is configured to provide imaging of the biological entities bound to the binding sites 112. The illumination system 118 may be configured to provide illumination patterns in relation to the binding sites 112 for enabling the information detected by the array of light-detecting areas 130 to provide spatial information of the biological entities arranged at the functionalized surface 110.

The information detected by the array of light-detecting areas 130 may provide a representation of the plurality of biological entities, without necessarily a visual image of the biological entities being formed. Data detected by the array of light-detecting areas 130 in one or more sequential illuminations of the plurality of biological entities may be processed in various manners. For instance, data processing may be performed to generate a multi-dimensional representation of the plurality of biological entities, which is not necessarily provided in form of a visual image. The multi-dimensional representation may for instance provide information of a sequence, molecular identity, morphology, or density of a biological entity based on acquired information, e.g., using a sequence of tags being provided to the plurality of biological entities.

The system 100 may further comprise a processing unit 146. The processing unit 146 may be configured to receive data detected by the array of light-detecting areas 130, e.g., via the circuitry 144. The processing unit 146 may be configured to process the received data for providing a representation of the biological entities that are bound at the binding sites 112. The processing unit 146 may for instance be configured to generate an image representation of the plurality of biological entities that are bound at the binding sites 112 or to generate sequencing of the biological entities.

The processing unit 146 may be implemented in hardware or as any combination of software and hardware. For instance, the processing unit 146 may be implemented in a common CPU or GPU with the controller 140 or in a separate CPU or GPU, wherein the CPU or GPU comprises software for providing functionality of the processing unit 146 in a general-purpose processor. Alternatively, the processing unit 146 may be implemented as firmware arranged in an embedded system on the common substrate 102. As a further alternative, the processing unit 146 may be implemented as a special-purpose circuitry for providing specific logical operations. Thus, the processing unit 146 may be provided in the form of an application-specific integrated circuit (ASIC), an application-specific instruction-set processor (ASIP) or a field-programmable gate array (FPGA).

According to yet another alternative, the data detected by the array of light-detecting areas 130 may be communicated to a processing unit 146 that may be arranged in a separate housing. Thus, the processing unit 146 may for instance be arranged in a server.

As mentioned above, the illumination waveguide 120 is configured to illuminate the biological entities that are bound at the binding sites 112. The illumination at the illumination areas 122 induces output of light that is associated with the biological entities. For instance, output of fluorescence light may be induced.

The biological entities may have inherent characteristics for interacting with the illumination light. For instance, the biological entities may be fluorescent. However, the system 100 may be configured to provide tags that may be configured to interact with the illumination light. For instance, the tags may comprise fluorophores for providing output of fluorescent light.

Each tag may for instance be configured to bind to a distinct molecule for identifying the molecule. In some embodiments, the tags may comprise a sequence of functional groups, such as nucleotides, configured to bind to a portion of the molecule having a complimentary sequence of functional groups.

A point in time of output light being induced and detected may provide information of the biological entity by using information of a particular tag being provided at the functionalized surface at that point in time.

In addition, output light associated with different biological entities may be differentiated by an emission wavelength of the output light, an intensity of the output light, and/or a lifetime of the output light. The emission wavelength of the output light may differ depending on characteristics of the biological entity or a tag binding to the biological entity. For instance, a fluorescence wavelength may differ depending on a fluorophore being excited by the illumination light. The intensity and lifetime of output light may also differ depending on characteristics of the biological entity or a tag binding to the biological entity.

The system 100 may be configured to provide time-gated lifetime detection of light by the light-detecting area 130. For instance, the circuitry 144 may be configured to read out detected light from the light-detecting area 130 using two or more time gates. This allows determination of a slope of detected light. Thus, a differentiation in lifetime of the output light may be detected.

Lifetime detection may be used as an alternative to detecting different wavelengths of light. Thus, using lifetime detection, use of a complex spectral filter for wavelength multiplexing may be avoided. The lifetime detection may be used for differentiating response between different tags providing different emission wavelength and different lifetime of emission.

The system 100 may be configured to sequentially transport different tags to the functionalized surface 110. Thus, different tags may be provided at different points in time. A tag being provided at the functionalized surface 110 may be washed away before the following tag in the sequence is provided to the functionalized surface 110.

Thus, the plurality of different tags may be provided sequentially in time, such that a response from the binding site 112 at a particular point in time could be associated with a particular tag for identifying the biological entity that is bound at the binding site 112. Hence, the response(s) from a single binding site 112 could be used for identifying the biological entity being bound at the binding site 112.

Further, different tags may be provided sequentially for binding to different parts of a biological entity. This may be used for sequencing of the biological entity. For instance, a tag may correspond to one base of the molecule.

The biological entity may be prepared such that a single tag may be added at a time to a sequence of the biological entity. Thus, tags may be sequentially added in an order along a sequence of the biological entity. For each tag being added, response to the illumination pattern may be detected from the binding sites 112. The response may be used for identifying the tag that is bound to the biological entity for determining information at a particular location within the sequence of the biological entity.

For instance, a fluorophore of the tag may block further nucleotides to be added to the sequence of the biological entity. In this manner, a sequence of the biological entity may be determined by determining one base at a time based on sequentially providing tags and removing the fluorophore after determining of the base and before providing a following tag.

In addition, a biological entity may be prepared by forming a plurality of fragments of the biological entity, which may be individually analyzed at binding sites 112. Information from the plurality of fragments may be used in combination for determining sequencing of the biological entity.

The processing unit 146 may be configured to process the received data for differentiating output light associated with different biological entities and/or different parts of biological entities. Thus, analysis of the biological entities that are bound at the binding sites 112 may be provided by processing of the received data.

In this regard, the processing unit 146 may use temporal information related to tag(s) being provided to the biological entities at the binding sites 112 at a point in time of detecting output light and spatial information related to illumination areas 122 at which output of light is induced. Hence, by using information of which type of tag that produced the output light and using information of a location that was illuminated by the illumination area 122, the processing unit 146 may be able to represent detailed information of the biological entities that are bound at the binding sites 112.

In particular, using a sequence of tags, the processing unit 146 may be configured to use temporal information and spatial information for processing the received data. The processing unit 146 may be configured to generate a reconstructed image of the biological sample.

The processing unit 146 may be configured to provide identification of biological entities and locations of the identified biological entities based on processing the detected responses, e.g., by taking the temporal information and the spatial information into account. The processing unit 146 may further determine the reconstructed image for representing a spatial distribution of different biological entities within the biological sample.

Thanks to the plurality of binding sites 112 of different binding site types being distributed across the functionalized surface 110, the processing unit 146 may be configured to reconstruct an image representing spatial relations between biological entities of a plurality of different categories within the biological sample.

According to an embodiment, the data acquired by the array of light-detecting areas 130 need not necessarily enable determining exact locations of the biological entities or binding sites 112 from which light is detected. The processing unit 146 may be configured to differentiate light output from biological entities bound at different binding sites 112 without necessarily being able to determine the exact location of the binding site 112. This may be used for associating information relating to the same biological entity and/or same binding site 112, which information is acquired in relation to different cycles in a sequence of providing tags. Thus, detailed information of a biological entity based on multiple tags binding to the biological entity in different cycles of a sequence of tags may be acquired without the exact location of the biological entity within a subset being necessarily determined.

According to an embodiment, the processing unit 146 is configured to generate one or more maps, wherein each map represents a spatial distribution of one or more biological entities in the biological sample.

The image reconstruction may thus provide a representation of the biological sample in form of maps of the biological sample representing one or more characteristics of the biological sample.

For instance, each map may represent a spatial distribution of a category of a biological entity or a particular biological entity within a category. The spatial distribution of a biological entity may be determined based on locations of the binding sites 112 at which the biological entity is bound, tags provided for inducing output of light that is associated with the biological entity, and selective illumination for illuminating the binding sites 112 such that the response from the biological entity may be differentiated from responses from other biological entities (that may be bound at other binding sites).

Each binding site 112 may be configured to bind a single biological entity. Thus, the processing unit 146 may be configured to process the received data to generate maps providing a physical count of biological entities. The presence of a biological entity at a binding site 112 corresponds to a single specimen of the biological entity being bound at the binding site 112.

The detected light in response to a plurality of illumination patterns may be processed to obtain a sample specific information data. The sample specific information data may comprise data representing detected light, such as data of intensity of light. Further, sample specific information data may comprise data representing a predefined term, such as identity of the biological entity, such as nucleotide, amino acid type etc.

According to one embodiment, raw data acquired in response to each illumination pattern are directly stitched together spatially to complete an information data matrix of the plurality of biological entities sequentially. This process may be repeated for multiple tags sequentially for providing the information data matrix with data related to multiple tags. Furthermore, the process may be repeated for several sequential steps of transporting different biological entities to the functionalized surface 110 and capturing the different biological entities at the binding sites 112.

According to another embodiment, the raw data acquired in response to each illumination may be pre-processed before obtaining of the sample specific information data. The pre-processing of raw data may take into account properties of the system 100, such as optical properties of the illumination waveguide 120, functionalized surface 110 and an optical path of light between the functionalized surface 110 and the array of light-detecting areas 130. For instance, a unit response function may be taken into account to deconvolute system related effects on the measurement. The properties of the system 100 may include intentional features such as illumination pattern and also imperfections due to fabrication.

For instance, the plurality of illumination patterns can be designed to increase frequency information acquired by the system 100 (e.g., through structured illumination). Then, a Fourier domain processing of multiple detections of light by the array of light-detecting areas 130 may be employed to obtain high frequency information from binding sites 112.

Further, the processing of data may also include steps to account for non-idealities of the illumination pattern due to imprecise light wave control or surface irregularities of the waveguide 120. A calibration method may be used to obtain information about such irregularities that is accounted for in the processing of the data. It is also foreseen that conventional data processing and Al based data processing methods may be employed to improve data accuracy such as through noise reduction.

Referring again to Fig. 2, the system 100 may further comprise an optical component between the array of light-detecting areas 130 and the waveguide 120.

For instance, the system 100 may comprise a filter 132 between the array of light-detecting areas 130 and the waveguide 120. The filter 132 may be configured to selectively transmit a wavelength or band of wavelengths of interest. This may be especially needed when the system 100 is used for detecting fluorescence light from the object. The filter 132 may thus be configured to transmit fluorescence light and reject excitation light, such that the excitation light does not reach the array of light-detecting areas 130.

The filter 132 may be formed from a material so as to selectively absorb or reflect light of undesired wavelengths, whereas light of interest from the object are passed by the filter 132.

However, the filter 132 may alternatively be formed by a plurality of layers 134, as illustrated in Fig. 2. The plurality of layers 134 may have appropriate thicknesses, such that light reflected back and forth through a layer will form constructive or destructive interference depending on the wavelength of light. Thus, the filter 132 may be arranged to selectively transmit light depending on wavelength. In an embodiment, the filter 132 may be formed as a Fabry-Pérot interference filter.

The filter 132 may be monolithically integrated on the array of light-detecting areas 130.

The system 100 may also or alternatively comprise an optical component configured to provide modulation of light for shaping output light towards the array of light-detecting areas 130. The optical component may thus be an optical modulator, which may be configured to provide shaping of the PSF.

The optical modulator may be arranged between the array of light-detecting areas 130 and the waveguide 120. The optical modulator may be provided between the array of light-detecting areas 130 and the filter 132 or may be provided between the filter 132 and the waveguide 120. The optical modulator may preferably be arranged sufficiently far away from the waveguide 120 as to avoid substantial scattering of the illumination light by the optical modulator.

The PSF is a projection of emission of a point source emitter at the functionalized surface 110 onto the array of light-detecting areas 130. By providing an optical modulator that may shape this projection locally, i.e., making the PSF spatially variant, additional information may be acquired and used in processing of the detected light. This additional information may be used for increasing resolution or throughput or read-out density, or for providing faster acquisition of information (i.e., using less frames to provide a desired representation of the biological entities).

Alternatively, the optical modulator may comprise a patterned optical layer, which could be used for generating a different PSF at different wavelengths. This may enable emission wavelength multiplexing. The patterned optical layer can be, but is not limited to, an optical metamaterial, photonic crystal, or optical antenna array.

The optical modulator may use an appropriate multilayer stack of a first type of layer and second type of layer with different refractive indices, wherein layers of the first type and layers of the second type are alternatingly provided in the multilayer stack, a type of interference filter can be constructed that responds differently for different angles at different wavelengths. This multilayer stack may be advantageously used in the system 100, when the array of light-detecting areas 130 is sufficiently dense and a sample arranged at the functionalized surface 110 is sufficiently sparse. Thus, the PSF emitted from the functionalized surface 110 may be provided to a plurality of light-detecting areas 130. However, using a sparse sample, different PSFs from different locations in the sample will not overlap at the array of light-detecting areas 130.

The differences in response of the optical modulator to different incident angles and different wavelengths may be sampled by read-out of the array of light-detecting areas 130. The processing unit 146 may be configured to use a suitable algorithm for identifying different emitters based on the detected responses, i.e., allowing wavelength multiplexing.

The processing of the received data detected by the array of light-detecting areas 130 may benefit from a sample being arranged in a pre-defined pattern on the functionalized surface 110. Alternatively, if the sample is not arranged in a pre-defined pattern, a distribution of the sample on the functionalized surface 110 could be measured and provided to the processing unit 146. This information puts a limit to an algorithm parameter space that accelerates imaging of the sample (less frames needed to reconstruct an image of the sample) and improves the image quality.

The pre-defined pattern could be a periodic pattern such as a square or hexagonal lattice, or a non-uniform pattern, or quasi-periodic lattice. Specifically, a non-uniform pattern may increase a condition number of an inverse problem for providing reconstruction of an image of the sample.

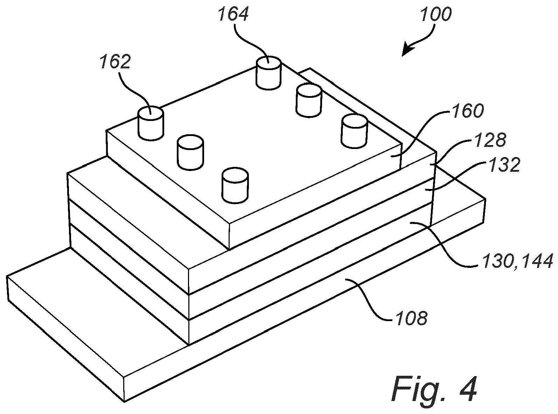

Referring now to Fig. 4, packaging of the system 100 described above into a compact device is described.

As indicated in Fig. 4, the system 100 described above may be provided in an integral device. The system 100 comprises an array of light-detecting areas 130 with associated read-out circuitry 144. The filter 132 is arranged above the array of light-detecting areas 130. In addition, a photonic integrated circuit (PIC) 128 including the waveguide 120 is arranged above the filter 132. In other words, the filter 132 is arranged between the PIC and the array of light-detecting areas 130.

The system 100 further comprises an electrical package 108. The electrical package 108 may comprise the controller 140 for controlling the PIC 128 and may also comprise the processing unit 146.

The system 100 may further comprise a microfluidic system 160. The microfluidic system 160 may be arranged above the PIC 128 with the functionalized surface 100 facing the microfluidic system 160. The microfluidic system 160 may be integrated with the PIC 128. For instance, the microfluidic system 160 may be bonded to the PIC 128.

The microfluidic system 160 may be configured to provide fluidic delivery to the functionalized surface 110. The microfluidic system 160 may be used for providing reagents to the functionalized surface 110 that may react with a sample and/or biological entities at the functionalized surface 110. The microfluidic system 160 may also or alternatively be configured to transport one or more tags to the functionalized surface 110. The tags may be used for tagging the biological entities by selectively binding to biological entities or parts of biological entities and providing output of light in response to being illuminated by the illumination pattern.

The microfluidic system 160 may be configured to sequentially transport different tags to the functionalized surface 110. The microfluidic system 160 may also be configured to transport buffers for washing away tags from the functionalized surface 110 before providing a following tag in the sequence to the functionalized surface 110.

The microfluidic system 160 may comprise inlet channels 162 and outlet channels 164 for providing input of fluids to the microfluidic system 160 and output of fluids from the microfluidic system 160.

As described above, illumination of the functionalized surface 110 may be provided by the illumination waveguide 120. Further, detection of output light is provided by the array of light-detecting areas 130. The microfluidic system 160 is provided at an opposite side of the functionalized surface 110 to the illumination waveguide 120 and the array of light-detecting areas 130. In this embodiment, no optical access to the functionalized surface 110 is necessary through the microfluidic system 160.

Thus, the microfluidic system 160 may be optically dense or opaque. This may reduce stray light at the functionalized surface 110 such that the output light induced by the illumination through the illumination waveguide 120 may be detected in relation to a low level of background noise. The microfluidic system 160 may be formed by silicon layers, polymers, glass, or other materials.

The illumination waveguide 120 may be formed from silicon nitride (SiN), which is suitable for transporting visible light, being essentially transparent to visible light, and is also suitable for monolithic integration on the array of light-detecting areas 130, since a layer of SiN may be formed without affecting an underlying semiconductor circuitry. Also, SiN has a refractive index facilitating total internal reflection in the illumination waveguide 110.

It should be realized that the illumination waveguide 120 may be formed by different materials. Also, the illumination waveguide 120 may be protected with an optically transparent protective layer facing the microfluidic system 160. The protective layer may provide a coating on the illumination waveguide and may provide resistance to liquids transported to the functionalized surface 110. For instance, the protective layer may protect the illumination waveguide from liquids used in sequencing chemistry.

For instance, the illumination waveguide 120 or the protective layer may be formed by tantalumpentoxide (Ta₂O₅), SiN, aluminum oxide (AlOx), or niobium oxide (NbOx), or other materials. The protective layer may provide patternable refractive index matching material to water.

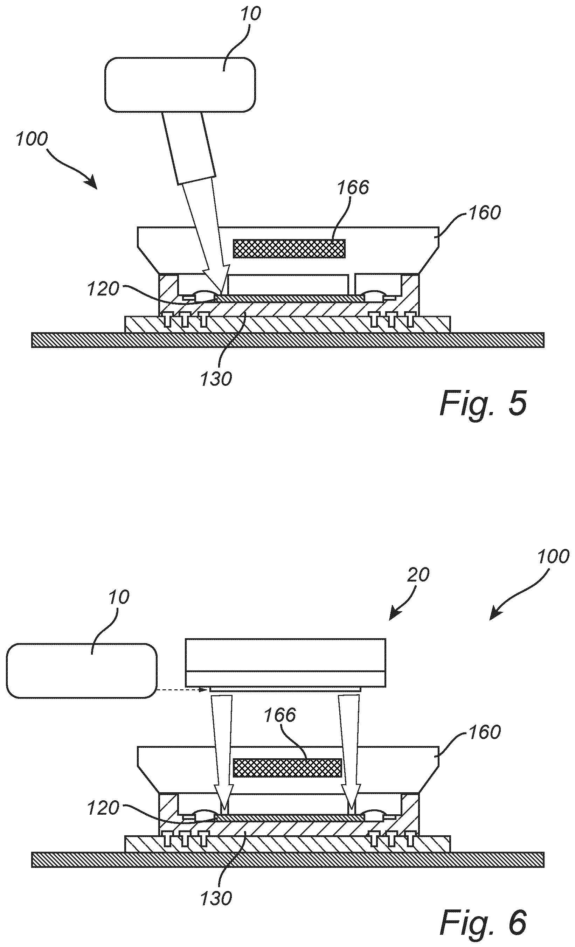

Referring now to Fig. 5, the system 100 is illustrated in relation to a light source 10. The light source 10 may be external to the system 100 and provide light to be introduced into the illumination waveguide 120. The light source 10 may provide coherent light. For instance, the light source may be a continuous wave laser light source.

The system 100 may be integrated in form of a cartridge that may be simple to mount in an instrument for analysis of biological entities. The instrument may include the light source 10.

As illustrated in Fig. 5, the system 100 may comprise a location in which light may be received from the light source 10. The system 100 may for instance comprise a light coupler for receiving light from the light source 10 into the system 100. The system 100 may further be configured to control the received light for controlling the illumination pattern formed in the illumination waveguide 120.

Only one or a few regions outside of the field of view (i.e., an area of the functionalized surface 110 from which output light is to be detected) can be used to couple the light into the system 100. A remaining surface of the cartridge can be covered by an opaque or optically dense functional layer, such as by an opaque or optically dense microfluidic system 160. As illustrated in Fig. 5, only a part 166 of the microfluidic system 160 above the functionalized surface 110 may be opaque.

Referring now to Fig. 6, the system 100 is illustrated in relation to an alternative manner of providing light into the system 100. In this case, light is provided from a light controlling unit 20, which may incorporate a light source or may receive light from a separate light source 10.

The light controlling unit 20 may provide active phase front modulation. Thus, the light controlling unit 20 may be configured to control a relation between one or more light waves that may be input into the illumination waveguide 120 of the system 100 from the light controlling unit 20.

Thanks to having a separate light controlling unit 20, the integrated system 100 may be simpler compared to the system 100 shown in Fig. 5. Thus, a simpler and cheaper consumable chip may be provided. In addition, a larger field of view may be provided.

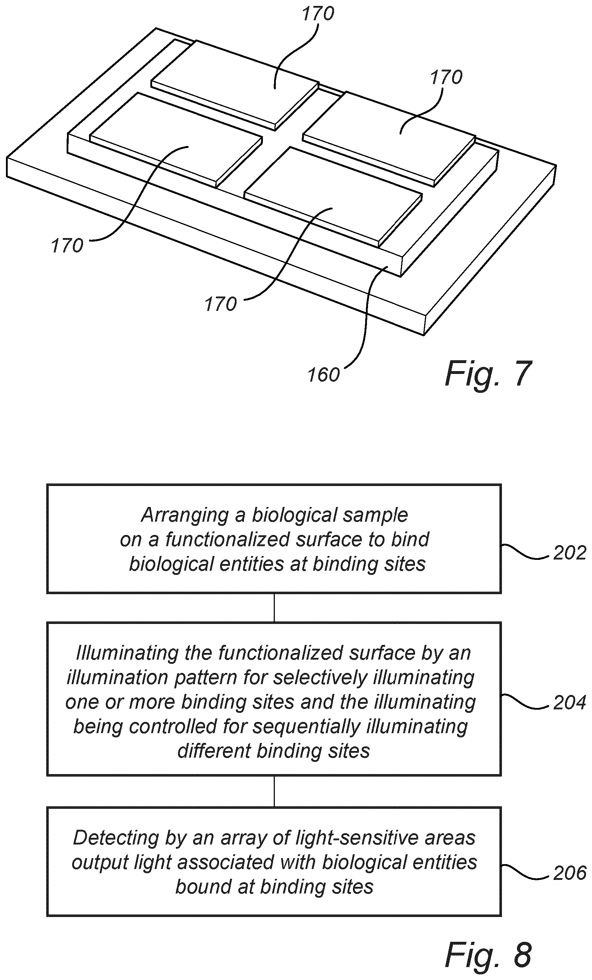

Referring now to Fig. 7, an embodiment of the system 100 for increased throughput is illustrated.

The functionalized surface 110, the illumination waveguide 120 and the array of light-detecting areas 130 may form an optical unit 170. The system 100 may comprise a plurality of optical units 170. This may provide a large, combined field of view for increased throughput.

Each optical unit 170 may thus comprise a separate illumination waveguide 120 for individually controlling the illumination pattern provided in respective optical units 170. Also, each optical unit 170 may comprise a separate array of light-detecting areas 130. Thus, a large total size of an area for light detection may be provided by using separate arrays.

The plurality of optical units 170 may be arranged in relation to a single microfluidic system 160. Thus, the plurality of optical units 170 may provide high throughput of acquiring information in relation to a single microfluidic system 160.

The system according to any of the embodiments described above may be part of an analysis system, which may for instance be configured to provide sequencing analysis of the biological entities. The analysis system may thus be referred to as a sequencing system.

Functions of the sequencing system may be realized by one or more entities in the sequencing system. For instance, the sequencing system may comprise a cartridge and an instrument entity. Each may comprise further sub-entities.

For instance, the cartridge may include a packaging, which may include electrical, optical, mechanical, thermal interfaces towards the instrument. The cartridge may further comprise CMOS chip, surface chemistry, and fluidics elements, including the microfluidic system 160. The fluidics elements may include local reservoirs, connectors, valves, filters, and also reagents.

The cartridge may also include auxiliary elements, such as a housing in which components are arranged, and connectors, e.g., for providing mechanical, electrical, and/or optical connection to the instrument.

For instance, the instrument may comprise an optical module, which may include lasers, couplers, fibers, lenses, mirrors, beam splitters, detectors, light modulators. The instrument may also include active and passive mechanical components to control the position of various entities. The instrument may include a fluidic module including reservoirs, tubing, valves, attachments, pumps, filters, connectors. The fluidic module of the instrument may be connected to the fluidics elements of the cartridge.

The instrument may include hardware and software units such as an FPGA, ASIC, CPU, GPU, volatile and non-volatile memory, network cards and peripherals for data routing, and data processing algorithms, e.g., data processing algorithms related to sequencing purposes.

A user of the sequencing system may expect specific performance metrics from the sequencing system pertinent to the sequencing of the biological entity. This may include accuracy, read-length, speed, read density and throughput. The design and optimization of the functional entities of the sequencing system may be configured to meet target performance metrics. The cartridge including one or more optical units 170 in different embodiments may be closely linked to other entities in the sequencing system for providing desired performance metrics.

According to an embodiment, the light source 10 is located in the instrument. The light emitted from the light source 10 is coupled to the system 100 to create illumination patterns. The light may be coupled to the PIC 128 via input ports on a cartridge.

A light beam from the light source 10 may be positioned with high accuracy (error < 10 micron), on the cartridge. Furthermore, this positioning ought to be kept during measurement process that can be minutes to days depending on the measurement. Environmental effects such as vibration, thermal fluctuations, mechanical shocks etc. can have detrimental effects on this requirement during a measurement. Any misalignments may lead to loss of optical power and thus fail the experiment prematurely. A beam positioner may be provided for controlling a location of the light beam in relation to the input port of the PIC 128. It is foreseen that an active feedback control loop may be implemented to ensure the positional accuracy between the light input port of PIC 128 and the light source 10.

According to an embodiment, a feedback control loop may be realized through a camera-based imaging system that continuously monitors the alignment of light beam to the input ports and provides optical feedback to the beam positioner to ensure acceptable optical power transfer to the PIC 128.

In an alternative embodiment, a detector in the system 100 can provide feedback to the beam positioner. The amount of light power in the PIC 128 may be monitored via dedicated light-detecting areas 130 in the optical unit 170. An alternative embodiment can utilize a secondary light detector monitoring the light power in the PIC 128 via a light output port dedicated to providing feedback to the beam positioner.

It should be realized that control of light beams may be adapted to a system 100 comprising a plurality of optical units 170, in which a plurality of light beams may be coupled to multiple PICs simultaneously. The same feedback loop may be used for observing relations of light beams to multiple PICs or dedicated loops may be created for each optical unit 170.

The optical units 170 may have direct fluidic interface with microfluidic distribution channels of the microfluidic system 160 in the cartridge. The microfluidic channels may be designed to ensure flow of fluids uniformly across the functionalized surface 110 at which the illumination patterns are formed.

Discrete optical units 170 may be positioned in parallel in relation to transport of fluids by the microfluidic system 160. In such case, the microfluidic system 160 may be configured to provide a parallelization of fluid flow, including splitting of the fluidic channels for transporting fluids to the plurality of optical units 170. Alternatively, the optical units 170 may be positioned in series, one after another, along a same microfluidic path of the microfluidic system 160.

According to one embodiment, the optical units 170 are located on a common side of a microfluidic path, defined by one or more fluidic channels. According to another embodiment, the optical units 170 are positioned on opposite sides of the microfluidic path. In such configuration, the same microfluidic path is in contact with multiple areas of illumination patterns, on opposite sides of the microfluidic path. This configuration provides an advantage of compactness as a same footprint can double a density of the binding sites 112.

According to an embodiment, the cartridge may comprise chemical or biological agents. The cartridge may comprise one or more reservoirs inside a housing of the cartridge. The reservoir(s) may host series of reagents such as washing fluids, hybridization reagents, tag reagents, cleavage reagents etc. Such reservoirs may be controlled with acoustic, fluidic, or mechanical forces to supply agents to the microfluidic system 160 in fluid form. It is also envisioned that such agents may be provided in solid form in the cartridge. The cartridge may be configured to provide a liquefaction process, which may turn the agents in solid form into liquid form, before the agents are used during experimentation.

According to another embodiment, the instrument may comprise chemical or biological agents in one or more reservoirs. This may enable larger quantities of consumable material to be stored for multiple experiments that run on a plurality of cartridges simultaneously or sequentially. The instrument may comprise one or more fluidic components, which may be configured to facilitate transfer of the agents to the cartridges. Thus, the one or more fluidic components may be, for instance, pumps, valves, tubes, mixers, robotic handlers, acoustic actuators, flow, and pressure sensors etc. During experiments, various agents may be transported in a mixture or pure liquid form to the cartridge for analyzing the biological entities in the cartridge.

The instrument may comprise a processing unit. The processing unit of the instrument may be responsible for processing vast amounts of data during processing of acquired data, such as for sequencing of biological entities. The processing unit of the instrument may be configured to control and acquire the data from the optical units 170, to process the raw data, to identify binding site information and translate acquired optical signals to sample data, such as identity determinations of the biological entities. Furthermore, the processing unit may also calculate quality scores to assess confidence in each identity determination.

As the amount of data generated during the measurements is vast, the processing unit may also perform data compression before data storage or data transfer via network to a remote server. The processing unit may also perform data file format changes and data encryption for secure and efficient data transfers. In an embodiment, the processing unit may also perform more advanced data analytics of bioinformatics such as base alignments, or variant calling. This is in particular useful in settings where viable network connection is not possible, such that data may not be easily transferred to an external unit, wherein such advanced data analytics could be performed.

The processing unit may be configured to manage sequencing protocols including mechanical, fluidic, optical, and electrical control and readouts.

The processing unit may be configured to control interacting with a user via an interface and may be configured to present information to the user and to receive information provided by the user. The processing unit may be configured to facilitate acquiring inputs from a user and providing real-time information to the user about status, errors, warnings and other auxiliary information about the system and progression of the measurement.

Different functions of the processing unit may require specialized hardware parts such as CPU, GPU, ASICS, FPGA in addition to memory and storage. Thus, the processing unit may be implemented in one or more hardware parts, possibly in combination with software instructions.

The processing unit may be configured to communicate with hardware specific controller units such as fluidic, electrical, mechanical modules to send analog and digital signals and read sensory outputs. The processing unit may be configured to establish communication protocols with such hardware specific controller units.

Referring now to Fig. 8, a method for analysis of a plurality of biological entities will be described.

The method comprises arranging 202 the biological sample on a functionalized surface. Before the biological sample is placed on the functionalized surface or when the biological sample is arranged on the functionalized surface, the biological sample may be interfaced with reagents for tissue permeabilization and binding biological entities at the binding sites.

The functionalized surface comprises a plurality of binding sites of a plurality of binding site types according to any of the embodiments described above. Each of the binding sites is configured to bind a biological entity from the biological sample. The binding sites of a binding site type are distributed across the plurality of binding sites and are configured to bind a biological entity from the biological sample. Binding sites of different binding site types are configured to bind different categories of biological entities from the biological sample. The plurality of binding sites comprises a plurality of subsets, wherein each subset comprises at least one binding site.

The method further comprises illuminating 204 the functionalized surface by an illumination pattern for selectively illuminating one or more binding sites at a time. The illuminating comprises controlling the illumination pattern for sequentially illuminating different binding sites and/or different parts of binding sites.

The method further comprises detecting 206, by an array of light-detecting areas, output light associated with biological entities bound at binding sites and illuminated by the illumination pattern, wherein each light-detecting area is associated with a subset of binding sites.

The detection of information based on selective illumination provides imaging of the biological entities being bound to the binding sites. The detected information may provide spatially resolved information which may be used for generating a visual representation of the imaging of the biological entities.

The method may further comprise transporting tags to the functionalized surface. Each tag may be configured to bind to a distinct type of the biological entities bound at binding sites. The illuminating of the functionalized surface by the illumination pattern may then induce output of light from the tags.

The method may further comprise determining an identification of the biological entities bound at the binding sites based on detecting of the output light from the tags.

The method may comprise sequentially transporting different tags to the functionalized surface and detecting output light from the different tags.

The method may further comprise processing data detected by the array of light-detecting areas. This may be used for reconstructing an image of the biological sample representing a spatial distribution of biological entities in the biological sample.

The method may further comprise binding distinct fluorescent-labeled tags to complementary portions of the biological entities.

The method may further comprise illuminating the fluorescent tags using an illumination pattern defined by interference of one or more light waves propagating in an illumination waveguide. The light waves may for instance be provided with different phase shifts for defining the illumination pattern by the interference of the light waves. The biological entities with tags may be illuminated in an evanescent field of light propagating in the illumination waveguide.

The method may further comprises detecting emitted light from the fluorescent labels via the array of light-detecting areas.

The method may further comprise repeating the illuminating and detecting steps using phase-tuning of the light waves for defining different illumination patterns. The different illumination patterns are defined to ensure complete illumination of the biological entities and detection of emitted light in response to the illumination. Thus, information is acquired from the entire biological entities using the provided tag in correspondence with spatial information defining the illumination of the entire biological entities.

The method may further comprise repeating the binding, illuminating, and detecting steps, using the repeated illuminating and detecting, with different types of fluorescent-labeled tags. In other words, a sequence of tags may be provided to the functionalized surface. The sequence may thus comprise a plurality of cycles, wherein each cycle corresponds to one occasion of providing tags to the functionalized surface. The tags may bind to the biological entities. While the tags are bound to the biological entities, sequential illumination of the binding sites using a plurality of illumination patterns and detection of responses may be performed. After the cycle is complete, tags may be removed, or at least a second portion of the tag interacting with illumination light may be removed, and new tags in the sequence of tags may be provided to the functionalized surface.

The different types of fluorescent-labeled tags may bind to different complementary portions of the biological entities for providing additional information relating to the biological entities. Thus, a sequence of different types of tags may be used for acquiring information of the biological entities. Information is acquired from the biological entities based on a specific tag in correspondence with temporal information defining a point in time during which the specific tag is provided to the biological entities. For sequencing of a molecule, the same type of tags may be provided in each cycle to allow determining information of a sequence of bases in the biological entities. The tags may be configured to control tags to only bind to the biological entities for forming a sequence of tags along the biological entity. This may ensure that an order of bases in the biological entity may be determined.

The method may further comprise determining the sequence of the biological entities, such as a sequence of a DNA molecule, an RNA molecule, a protein, a metabolite, a lipid, a sugar, or another molecular feature, based on detected light from the array of light-detecting areas using the temporal information and the spatial information. As an alternative, the method may comprise determining a distinctive pattern within a sequence enabling unique identification of a molecule, such as protein or peptide fingerprint.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100) for spatial analysis of a biological sample, said system comprising:
a functionalized surface (110), wherein the functionalized surface (110) is configured to receive the biological sample and comprises a plurality of binding sites (112) of a plurality of binding site types, wherein the binding sites (112) of a binding site type are distributed across the plurality of binding sites (112) and are configured to bind a biological entity from the biological sample, wherein binding sites (112a-d) of different binding site types are configured to bind different categories of biological entities from the biological sample, wherein the plurality of binding sites (112) comprises a plurality of subsets (114), wherein each subset (114) comprises at least one binding site (112) or part of a binding site (112);
an illumination system (118) arranged in relation to the functionalized surface (110) and configured to form an illumination pattern at the functionalized surface (110) for selectively illuminating one or more binding sites (112) at a time; and
an array of light-detecting areas (130) arranged in relation to the functionalized surface (110) and configured to detect output light associated with biological entities bound at binding sites (112) and illuminated by the illumination pattern, wherein each light-detecting area (130) is associated with a subset (114) of the binding sites (112);
wherein the illumination system (118) and the array of light-detecting areas (130) are configured to provide imaging of the functionalized surface (110) for differentiating responses to the illumination pattern from different binding sites (112).

2. The system according to claim 1, wherein each subset (114) comprises a plurality of binding sites (112) of each binding site type.

3. The system according to claim 2, wherein the illumination system (118) is configured to form the illumination pattern comprising one or more illumination areas (122), wherein each illumination area (122) illuminating a single binding site (112) of a same binding site type in the subset at a time.

4. The system according to any one of the preceding claims, wherein the illumination system (118) is configured to form the illumination pattern comprising more than one illumination area (122), wherein each illumination area (122) is associated with one or more unique subsets (114).

5. The system according to any one of the preceding claims, wherein the illumination system (118) comprises an illumination waveguide (120) integrated with the functionalized surface (110) and configured to propagate one or more light waves for forming the illumination pattern based on the one or more light waves, wherein the illumination system (118) further comprises a controller (140) which is configured to control the one or more light waves propagating in the illumination waveguide (120) for sequentially illuminating different binding sites (112).

6. The system according to claim 5, wherein the array of light-detecting areas (130) is integrated with the illumination waveguide (120).

7. The system according to any one of the preceding claims, wherein a size of each of the binding sites (112) is smaller than 1000 nm, such as in a range of 1 - 750 nm, such as in a range of 5 - 500 nm, and an extension of an illumination area (122) in the illumination pattern is smaller than 10 µm, such as in a range of 100 nm - 10 µm.

8. The system according to any one of the preceding claims, wherein the illumination system (118) is configured to illuminate the biological entities bound at binding sites (112) for inducing the output light associated with the biological entities, such as fluorescence light, wherein the system (100) is configured to differentiate output light associated with different biological entities bound by different binding sites (112) by at least one of: a point in time at which the output light is induced, an emission wavelength of the output light, an intensity of the output light, or a lifetime of the output light.

9. The system according to any one of the preceding claims, wherein the illumination system (118) is configured to form the illumination pattern for selectively illuminating the binding site by an evanescent field and configured to induce output of fluorescence light.

10. The system according to any one of the preceding claims, further comprising a microfluidic system (160) configured to transport one or more tags to the functionalized surface (110) for tagging the biological entities bound at binding sites (112) and for providing output of light from the biological entities illuminated by the illumination pattern.

11. The system according to any one of the preceding claims, further comprising a processing unit (146) configured to receive data detected by the array of light-detecting areas (130), wherein the processing unit (146) is configured to generate a reconstructed image of the biological sample based on the received data.

12. The system according to any one of the preceding claims, wherein the functionalized surface (110) comprises at least one antifouling region (116) configured to reduce an adsorption speed or prohibit adsorption of undesired biological entities at the plurality of binding sites (112), wherein the at least one antifouling region (116) is arranged surrounding one or more binding sites (112).

13. A method for spatial analysis of a biological sample, said method comprising:
arranging (202) the biological sample on a functionalized surface, wherein the functionalized surface comprises a plurality of binding sites of a plurality of binding site types, wherein the binding sites of a binding site type are distributed across the plurality of binding sites and are configured to bind a biological entity from the biological sample, wherein binding sites of different binding site types are configured to bind different categories of biological entities from the biological sample, wherein the plurality of binding sites comprises a plurality of subsets, wherein each subset comprises at least one binding site or part of a binding site;
illuminating (204) the functionalized surface by an illumination pattern for selectively illuminating one or more binding sites at a time, wherein the illuminating comprises controlling the illumination pattern for sequentially illuminating different binding sites;
detecting (206), by an array of light-detecting areas, output light associated with biological entities bound at binding sites and illuminated by the illumination pattern, wherein each light-detecting area is associated with a subset of the binding sites, and wherein the illuminating and detecting provides imaging of the functionalized surface for differentiating responses to the illumination pattern from different binding sites.

14. The method according to claim 13, further comprising:
transporting tags to the functionalized surface, wherein each tag is configured to bind to a distinct type of the biological entities bound at binding sites, wherein said illuminating of the functionalized surface by the illumination pattern induces output of light from the tags; and
determining an identification of the biological entities bound at the binding sites based on detecting of the output light from the tags.

15. The method according to claim 14, further comprising reconstructing an image of the biological sample representing a spatial distribution of presence and identifications of the biological entities in the biological sample.
